(19) Europäisches Patentamt
European Patent Office
Office européen des brevets

(11) **EP 3 738 624 B1**

(12) **EUROPEAN PATENT SPECIFICATION**

(45) Date of publication and mention
of the grant of the patent:
**14.09.2022 Bulletin 2022/37**

(21) Application number: **18900480.7**

(22) Date of filing: **27.12.2018**

(51) International Patent Classification (IPC):
***A61M 1/16*** *(2006.01)*

(52) Cooperative Patent Classification (CPC):
**A61M 1/1609; A61M 1/1603;** A61M 2205/50;
A61M 2205/52; A61M 2230/20; A61M 2230/207

(86) International application number:
**PCT/JP2018/048346**

(87) International publication number:
**WO 2019/138917 (18.07.2019 Gazette 2019/29)**

(54) **APPARATUS FOR CALCULATING AMOUNT OF EXTRACELLULAR FLUID AND METHOD FOR CALCULATING AMOUNT OF EXTRACELLULAR FLUID**

VORRICHTUNG ZUR BERECHNUNG DER MENGE AN EXTRAZELLULÄRER FLÜSSIGKEIT UND VERFAHREN ZUR BERECHNUNG DER MENGE AN EXTRAZELLULÄRER FLÜSSIGKEIT

APPAREIL DE CALCUL DE QUANTITÉ DE FLUIDE EXTRACELLULAIRE ET PROCÉDÉ DE CALCUL DE QUANTITÉ DE FLUIDE EXTRACELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.01.2018 JP 2018002171**

(43) Date of publication of application:
**18.11.2020 Bulletin 2020/47**

(73) Proprietor: **Nipro Corporation
Osaka-shi, Osaka 531-8510 (JP)**

(72) Inventors:
- **SHINZATO, Toru**
  **Toyohashi-shi, Aichi 441-8107 (JP)**
- **MIWA, Masamiki**
  **Kitanagoya-shi, Aichi 481-0042 (JP)**

(74) Representative: **Söllner, Udo
Patentanwalt
Freundorferstrasse 34c
85598 Baldham (DE)**

(56) References cited:
**EP-A1- 2 594 300     JP-A- S57 183 855**

**Description**

Technical Field

The technique disclosed herein relates to calculation of extracellular fluid volume.

Background Art

Since kidneys malfunction in hemodialysis patients, all of the water they take in accumulates in their bodies. When the accumulated water in the bodies is removed by a hemodialysis procedure, the water is removed until a water volume in extracellular compartment (which may be referred to as an extracellular fluid volume, hereinbelow) becomes theoretically equal to an extracellular fluid volume of persons with normally functioning kidneys. In fact, however, a method for measuring an extracellular fluid volume has not been established. Thus, at present, a body weight of a patient whose extracellular fluid volume is estimated to be equal to that of a person with normally functioning kidneys is defined as a dry weight, and water in the body is removed until the patient's body weight becomes equal to the dry weight (Luik A, et al: Blood pressure control and fluid state in patients on long treatment time dialysis. J Am Soc Nephrol 5: 521, 1994). The dry weight is determined through a trial and error process based on clinical symptoms such as whether edema is observed or not, blood pressure level, whether a drop in blood pressure is observed during hemodialysis or not, whether the patient feels fatigued after hemodialysis or not, whether a muscle cramp is observed in a time window from a latter part of hemodialysis to post-hemodialysis or not, and the like (Jaeger JQ and Mehta RL: Assessment of Dry Weight in Hemodialysis: An Overview. J Am Soc Nephrol 10: 392-403, 1999). In fact, however, evaluation results on such clinical symptoms may be incorrect (Charra B, et al: Clinical assessment of dry weight. Nephrol Dial Transplant 11 (Suppl 2): 16-19, 1996). Further, since body fat amount and/or muscle mass change(s) as some time elapses, the determined dry weight may not be used for a long time (Jaeger JQ and Mehta RL: Assessment of Dry Weight in Hemodialysis: An Overview. J Am Soc Nephrol 10: 392-403,1999).

At present, one of methods for assessing whether a patient's post-hemodialysis weight is equal to his/her dry weight is to check if edema occurs or not. It is considered that edema occurs when an extracellular fluid volume of a patient is greater by 3 to 5 kg than that of a person with normally functioning kidneys (Gunal AI: How to determine 'dry weight'? Kidney Int 3: 377-379, 2013). This means that even if the extracellular fluid volume of patient at the set dry weight is greater than that of the person with normally functioning kidneys, it is considered that edema is not occurring if the difference is equal to or less than 3 to 5 kg. Thus, determining whether the dry weight is too low or not based on the presence/absence of edema may result in that the dry weight is set higher than it should be. When water is excessively accumulated in the body, volume of blood, which is a part of extracellular fluid, also increases and the heart is thereby enlarged. In view of this, another method for assessing whether the dry weight is appropriate or not is to make an assessment based on the size of heart relative to the rib cage (cardiothoracic ratio) in a chest X-ray radiograph. When a cardiothoracic ratio in a chest X-ray radiograph taken after hemodialysis is approximately 50%, the extracellular fluid volume is determined as appropriate (Gunal AI: How to determine 'dry weight'? Kidney Int 3: 377-379, 2013). Further, it is known that atrial natriuretic peptide (which will be referred to as hANP hereinbelow) is secreted in large quantity when the heart is strained. In view of this, a hANP concentration in post-hemodialysis blood is measured, and when it is an appropriate concentration (40 to 60 pg/mL), the dry weight is determined as appropriate (Eriko ISHII, et al.: The target range of plasma ANP level for dry weight adjustment in HD patients, Journal of Japanese Society for Dialysis Therapy, 37:1417-1422, 2004). However, in patients with cardiac failure and/or cardiac valvular disease, the hANP concentration and/or the cardiothoracic ratio increases even though the extracellular fluid volume is appropriate (Brandt RR et al: Atrial natriuretic peptide in heart failure. J Am Coll CardioL 22 (4 Suppl A): 86A-92A, 1993). Here, hemodialysis patients have a high probability of getting cardiac failure and/or cardiac valvular disease.

Relevant prior art is for instance disclosed in document EP2594300 A1.

Summary of Invention

Technical Problem

In a hemodialysis patient, water has excessively accumulated in extracellular compartment before hemodialysis. Thus, the water is removed during hemodialysis until the extracellular fluid volume of the patient becomes equal to that of a person with normally functioning kidneys. That is, the water is removed during hemodialysis until the patient's weight becomes his/her dry weight.

The extracellular fluid volume, based on which the dry weight is determined, is estimated based on clinical symptoms. However, evaluation results of the clinical symptoms may often be incorrect. Thus, even though the water is removed during hemodialysis until the patient's weight becomes equal to the dry weight, the post-hemodialysis extracellular fluid volume may not always be appropriate actually.

Further, fat amount and/or muscle mass of a hemodialysis patient change depending on his/her nutritional condition. The patient's extracellular fluid volume changes as the fat amount and/or muscle mass change. Thus, the dry weight needs to be updated regularly. However, conventional methods for determining dry weight have accuracy issues and are not suitable for frequently resetting dry weight to keep the extracellular fluid volume at appropriate level. For example, a method for assessing an extracellular fluid volume based on whether edema is observed or not estimates the extracellular fluid volume of a hemodialysis patient based on the patient's appearance, which may make the assessment difficult due to the skin condition. Further, since edema does not occur unless the extracellular fluid volume becomes greater by at least 3 to 5 kg than the appropriate extracellular fluid volume (Gunal AI: How to determine 'dry weight'? Kidney Int 3: 377-379, 2013), the method based on whether edema is observed or not can detect abnormality in the extracellular fluid volume only when the extracellular fluid volume is significantly increased. Furthermore, the assessment may differ depending on skills of assessors (e.g., doctors), thus it cannot be said that the assessment is always correct. Another method for assessing an extracellular fluid volume based on a chest X-ray radiograph requires time and labor to take a chest X-ray radiograph and also requires a facility for taking X-ray radiographs. Further, a hemodialysis patient with cardiac depression, that is, a hemodialysis patient with cardiac failure and/or cardiac valvular disease, may have a large cardiothoracic ratio even though the dry weight is appropriate, that is, even though the extracellular fluid volume is appropriate. In other words, the cardiothoracic ratio is not reliable when the patient has cardiac failure and/or cardiac valvular disease. Another method for assessing an extracellular fluid volume from a hANP concentration costs significantly for measuring a hANP concentration and is not suitable to be frequently carried out. Further, a hemodialysis patient with cardiac depression, that is, a hemodialysis patient with cardiac failure and/or cardiac valvular disease, may have a high hANP concentration even though the extracellular fluid volume is appropriate. Thus, for the hemodialysis patient with a cardiac disease, whether his/her extracellular fluid volume is appropriate or not cannot be accurately determined based on chest X-ray radiograph or hANP concentration.

The disclosure herein discloses a technique that assesses an extracellular fluid volume of a hemodialysis patient accurately and easily.

Solution to Technical Problem

An extracellular fluid volume calculator according to the present invention comprises the technical features of independent claim 1. A method for calculating an extracellular fluid volume according to the present invention comprises the technical features of independent claim 7.

A first extracellular fluid volume calculator disclosed herein may comprise: a first acquirement unit configured to acquire a pre-hemodialysis plasma uric acid concentration, a post-hemodialysis plasma uric acid concentration, a removal amount of uric acid by hemodialysis, and a removal volume of water during hemodialysis; and a first processor configured to calculate an extracellular fluid volume based on a difference between a pre-hemodialysis amount of uric acid and a post-hemodialysis amount of uric acid, the difference being determined based on the pre-hemodialysis plasma uric acid concentration, the post-hemodialysis plasma uric acid concentration, the removal amount of uric acid, and the removal volume of water acquired by the first acquirement unit.

The above extracellular fluid volume calculator calculates a post-hemodialysis extracellular fluid volume, focusing on the difference between the pre-hemodialysis amount of uric acid and the post-hemodialysis amount of uric acid in an extracellular compartment. Since the calculator does not estimate the post-hemodialysis extracellular fluid volume but calculates the post-hemodialysis extracellular fluid volume based on the actually measured elements, whether the post-hemodialysis extracellular fluid volume is appropriate or not can be accurately assessed. Further, the pre-hemodialysis plasma uric acid concentration, the post-hemodialysis plasma uric acid concentration, and the removal volume of water by hemodialysis can be easily acquired with few additional, special procedure, thus the post-hemodialysis extracellular fluid volume can be assessed easily.

A method for calculating an extracellular fluid volume may comprise: a first acquirement step of acquiring a pre-hemodialysis plasma uric acid concentration; a second acquirement step of acquiring a post-hemodialysis plasma uric acid concentration; a third acquirement step of acquiring a removal amount of uric acid by hemodialysis and a removal volume of water by hemodialysis; and a calculation step of calculating a post-hemodialysis extracellular fluid volume based on a difference between a pre-hemodialysis amount of uric acid and a post-hemodialysis amount of uric acid in an extracellular compartment, the balance being determined based on the pre-hemodialysis plasma uric acid concentration, the post-hemodialysis plasma uric acid concentration, the removal amount of uric acid, and the removal volume of water acquired in the first, second, and third acquirement steps.

The above method for calculating an extracellular fluid volume calculates a post-hemodialysis extracellular fluid volume, focusing on the difference in uric acid quantities. Thus, whether the post-hemodialysis extracellular fluid volume in the body is appropriate or not can be assessed accurately and easily.

A second extracellular fluid volume calculator disclosed herein may comprise: a first acquirement unit configured to acquire a pre-hemodialysis substance concentration, a post-hemodialysis substance concentration, a removal amount of substance by hemodialysis, and a removal volume of water during hemodialysis, wherein the pre-hemodialysis substance concentration is a pre-hemodialysis concentration of a substance in plasma, the post-hemodialysis substance concentration is a post-hemodialysis concentration of the substance in plasma, and the substance is incapable of passing through a cell membrane and is capable of passing through a capillary membrane; and a first processor configured to calculate an extracellular fluid volume based on a difference between a pre-hemodialysis amount of the substance and a post-hemodialysis amount of the substance, the difference being determined based on the pre-hemodialysis substance concentration, the post-hemodialysis substance concentration, the removal amount of substance, and the removal volume of water acquired by the first acquirement unit.

The above extracellular fluid volume calculator calculates a post-hemodialysis extracellular fluid volume, focusing on the substance that fulfills the three conditions: it is incapable of passing through a cell membrane, capable of passing through a capillary membrane, and eliminable by hemodialysis. Thus, this extracellular fluid volume calculator can bring the same effects as the first extracellular fluid volume calculator.

Brief Description of Drawings

FIG. 1 shows a system configuration of an extracellular fluid volume calculator according to an embodiment.
FIG. 2 schematically shows substances in extracellular and intracellular compartments.
FIG. 3 shows a flowchart of an exemplary process for a processor to calculate an extracellular fluid volume.
FIG. 4 shows a relationship between normalized intracellular fluid volume and normalized extracellular fluid volume.
FIG. 5 shows corrected post-hemodialysis normalized extracellular fluid volume (corrected nECVe).
FIG. 6 shows monthly transition in post-hemodialysis normalized intracellular fluid volume in a hemodialysis patient with a diabetic disease.
FIG. 7 shows a relationship between calculated post-hemodialysis hematocrit values and measured post-hemodialysis hematocrit values.

Description of Embodiments

Some of the features characteristic to below-described embodiments will herein be listed. It should be noted that the respective technical elements are independent of one another, and are useful solely or in combinations.

(Feature 1) The extracellular fluid volume calculator disclosed herein can easily acquire a pre-hemodialysis plasma uric acid concentration and a post-hemodialysis plasma uric acid concentration by blood samples being taken from a hemodialysis patient before and after hemodialysis. Thus, the extracellular fluid volume calculator can calculate an extracellular fluid volume with few additional, special procedure and without significant cost.

(Feature 2) In the extracellular fluid volume calculator disclosed herein, the acquirement unit may be configured to further acquire a pre-hemodialysis hematocrit value, a post-hemodialysis hematocrit value, a hemodialysis period, a blood flow rate passing through a dialyzer during hemodialysis, a dialysate flow rate passing through the dialyzer during hemodialysis, and a dialyzer overall mass transfer-area coefficient for uric acid used in hemodialysis. The processor may be configured to: calculate a plasma flow rate based on the pre-hemodialysis hematocrit value, the post-hemodialysis hematocrit value, and the blood flow rate acquired by the acquirement unit; calculate a dialyzer clearance for uric acid used in hemodialysis based on the dialyzer overall mass transfer-area coefficient for uric acid and the dialysate flow rate passing through the dialyzer during hemodialysis acquired by the acquisition unit and the calculated plasma flow rate; and calculate the removal amount of uric acid based on the pre-hemodialysis plasma uric acid concentration, the post-hemodialysis plasma uric acid concentration, and the hemodialysis period acquired by the acquirement unit and the calculated dialyzer clearance for uric acid. This configuration enables the removal amount of uric acid to be calculated from numerical values that can be easily obtained from the blood of hemodialysis patient before and after hemodialysis and easily obtainable numerical values regarding hemodialysis. Thus, the extracellular fluid volume calculator can calculate a post-hemodialysis extracellular fluid volume with few additional, special procedure and without significant cost.

(Feature 3) In the extracellular fluid volume calculator disclosed herein, the acquirement unit may be configured to further acquire a pre-hemodialysis body weight and a post-hemodialysis body weight. The processor may be configured to calculate the removal volume of water from the pre-hemodialysis body weight and the post-hemodialysis body weight acquired by the acquirement unit. This configuration enables a post-hemodialysis extracellular fluid volume to be calculated easily with few additional, special procedure and without significant cost.

(Feature 4) In the extracellular fluid volume calculator disclosed herein, the acquirement unit may be configured to further acquire a body height. The processor may be configured to calculate an ideal body weight from the body height acquired by the acquirement unit, and convert each of the calculated extracellular fluid volume and intracellular fluid volume into a volume per kilogram of the ideal body weight. This configuration enables the extracellular fluid volume to be assessed as a volume per kilogram of the ideal body weight, thus it can alleviate an influence of body shape difference due to fat amount difference among hemodialysis patients, and more accurately assess whether the extracellular fluid volume is appropriate or not.

(Feature 5) In the extracellular fluid volume calculator disclosed herein, the acquirement unit may be configured to further acquire a pre-hemodialysis plasma urea concentration, where urea is a substance that is distributed in all body fluids and passes through cell membranes, a post-hemodialysis plasma urea concentration, and a removal amount of urea by hemodialysis. The processor may be configured to: calculate a total body fluid volume based on a difference between pre-hemodialysis and post-hemodialysis urea quantities in all body fluids that is determined based on the pre-hemodialysis plasma urea concentration, the post-hemodialysis plasma urea concentration, the removal amount of urea, and the removal volume of water acquired by the acquirement unit; calculate an intracellular fluid volume from the calculated total body fluid volume and extracellular fluid volume; and correct the extracellular fluid volume with the calculated intracellular fluid volume. Since urea distributes in all body fluids, this configuration can calculate the total body fluid volume, focusing on the difference between the pre-hemodialysis and post-hemodialysis urea quantities. Further, the configuration can calculate the intracellular fluid volume from the calculated total body fluid volume and extracellular fluid volume. It is known that an intracellular fluid volume changes depending on muscle mass. When the muscle mass changes, the extracellular fluid volume also changes by an amount corresponding to the muscle mass change. Here, the change in extracellular fluid volume corresponding to the change in muscle mass does not affect circulatory organs. Thus, by correcting the extracellular fluid volume with the intracellular fluid volume, a part of the extracellular fluid volume that changed as the muscle mass changed and does not affect circulatory organs can be cancelled out. Accordingly, correcting the extracellular fluid volume with the intracellular fluid volume can alleviate an influence of the muscle mass difference and accurately assess whether or not the extracellular fluid volume is great enough to excessively burden the circulatory organs.

(Feature 6) In the extracellular fluid volume calculator disclosed herein, the substance used for calculating the total body fluid volume is urea. This configuration facilitates the calculation of the total body fluid volume since the dialyzer overall mass transfer-area coefficient for urea, the pre-hemodialysis plasma urea concentration, and the post-hemodialysis plasma urea concentration can be easily obtained.

Embodiment

Hereinbelow, an extracellular fluid volume calculator 10 according to an embodiment will be described. The extracellular fluid volume calculator 10 is configured to calculate an extracellular fluid volume in the body of a hemodialysis patient. It is known that even when a water volume in a hemodialysis patient's body becomes excessive (i.e., the patient becomes overhydrated) or becomes insufficient (i.e., the patient becomes underhydrated), a water volume in an intracellular compartment 40 hardly changes and only a water volume in an extracellular compartment 50 changes. Thus, it is an extracellular fluid volume that is needed to be adjusted by water removal through hemodialysis. In order to assess whether a post-hemodialysis extracellular fluid volume of the hemodialysis patient is appropriate or not, the extracellular fluid volume calculator 10 according to the present embodiment is configured to calculate the post-hemodialysis extracellular fluid volume of the hemodialysis patient.

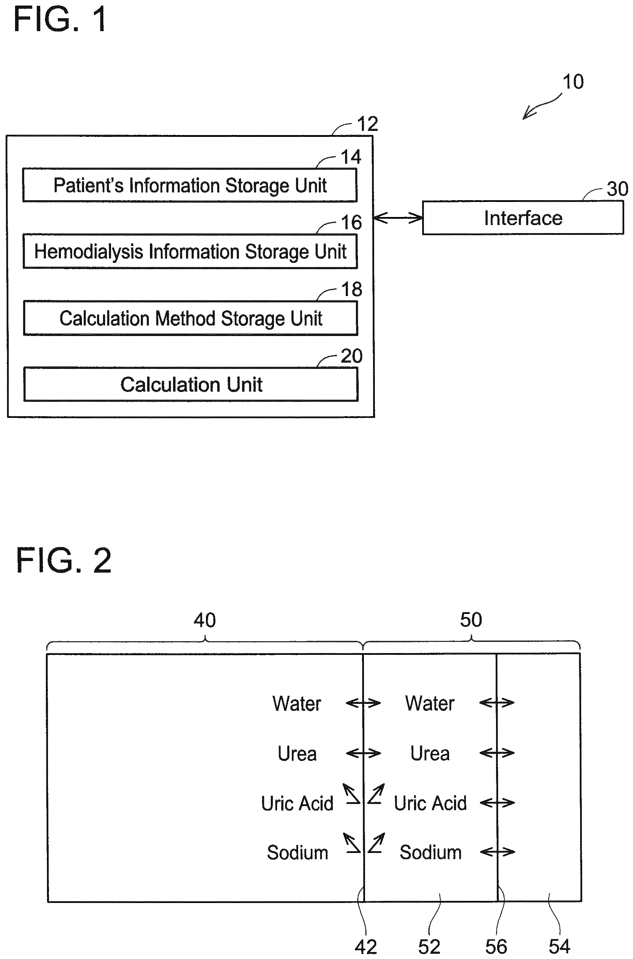

As shown in FIG. 1, the extracellular fluid volume calculator 10 includes a processor 12 and an interface 30. The processor 12 may be configured of a computer including a CPU, a ROM, a RAM, etc., for example. The processor 12 functions as a calculation unit 20 shown in FIG. 1 by the computer executing a program. A process executed by the calculation unit 20 will be described later in detail. The calculation unit 20 is an example of "processor".

As shown in FIG. 1, the processor 12 further includes a patient's information storage unit 14, a hemodialysis information storage unit 16, and a calculation method storage unit 18. The patient's information storage unit 14 is configured to store various kinds of information about the hemodialysis patient. The patient's information storage unit 14 stores information about the hemodialysis patient inputted through the interface 30 and information about the hemodialysis patient calculated by the calculation unit 20. The information about the hemodialysis patient inputted through the interface 30 includes pre-hemodialysis and post-hemodialysis plasma uric acid concentrations, pre-hemodialysis and post-hemodialysis uric concentrations, pre-hemodialysis and post-hemodialysis serum sodium concentrations, pre-hemodialysis and post-hemodialysis hematocrit values, and the body height of the hemodialysis patient, for example. The information about the hemodialysis patient calculated by the calculation unit 20 includes a post-hemodialysis extracellular fluid volume, a post-hemodialysis total body fluid volume, and a post-hemodialysis intracellular fluid volume that are calculated based on the information inputted through the interface 30, for example.

The hemodialysis information storage unit 16 is configured to store various types of information about hemodialysis. The hemodialysis information storage unit 16 stores information about hemodialysis inputted through the interface 30 and information about hemodialysis calculated by the calculation unit 20. The information about hemodialysis inputted through the interface 30 includes a removal volume of water by hemodialysis, a hemodialysis period, blood and dialysate flow rates passing through a dialyzer during hemodialysis, and the value of dialyzer overall mass transfer-area coefficient for urea used in hemodialysis which is described in its catalogue, for example. The information about hemodialysis calculated by the calculation unit 20 includes a removal amount of uric acid that are calculated based on the information inputted through the interface 30, for example.

The calculation method storage unit 18 is configured to store various mathematical formulas used for calculating a post-hemodialysis extracellular fluid volume. For example, the calculation method storage unit 18 stores formulas of Mathematical 3, 12, 14, 20, 23 to 26, 28, 30, 38 to 41, and 43 which will be described later in detail. The calculation unit 20 is configured to calculate various numerical values that are used for calculating post-hemodialysis extracellular fluid volume, and post-hemodialysis intracellular fluid volume, by substituting the various numerical values stored in the patient's information storage unit 14 and the hemodialysis information storage unit 16 into the formulas stored in the calculation method storage unit 18.

The interface 30 is a display device configured to provide (output) various types of information calculated by the extracellular fluid volume calculator 10 to an operator, and is also an input device configured to receive instructions and information from the operator. The interface 30 can display, to the operator, a calculated post-hemodialysis extracellular fluid volume, a calculated post-hemodialysis intracellular fluid volume, a post-hemodialysis normalized extracellular fluid volume corrected with a post-hemodialysis intracellular fluid volume normalized by an ideal body weight or the post-hemodialysis intracellular fluid volume, and the like, for example. Further, the interface 30 can receive input of various types of information about the hemodialysis patient (pre-hemodialysis and post-hemodialysis plasma uric acid concentrations, pre-hemodialysis and post-hemodialysis plasma uric concentrations, pre-hemodialysis and post-hemodialysis serum sodium concentrations, pre-hemodialysis and post-hemodialysis hematocrit values, a body height, etc.) and various types of information about hemodialysis (removal volume of water, hemodialysis period, blood flow rate passing through a dialyzer, dialysate flow rate passing through the dialyzer, the value of dialyzer overall mass transfer-area coefficient for urea which is described in its catalogue, etc.). The interface 30 is an example of "acquirement unit".

Here, a method for calculating a post-hemodialysis extracellular fluid volume using various types of information inputted to the interface 30 will be described. The extracellular fluid volume calculator 10 according to the present embodiment is configured to calculate a post-hemodialysis extracellular fluid volume, focusing on the difference between pre-hemodialysis and post-hemodialysis uric acid quantities in the extracellular compartment 50. As shown in FIG. 2, a fluid compartment in a body is divided into an intracellular compartment 40 and an extracellular compartment 50, and the extracellular compartment 50 is divided into an interstitial compartment 52 and an intravascular compartment 54. Uric acid is distributed in both the intracellular compartment 40 and the extracellular compartment 50, and substantially does not pass through cell membranes within four hours or so, which is a typical hemodialysis period. During hemodialysis, uric acid does not pass through a cell membrane 42 but passes through a capillary membrane 56, thus the post-hemodialysis extracellular fluid volume can be calculated by focusing on the difference between pre-hemodialysis and post-hemodialysis uric acid quantities in the extracellular compartment 50.

A method for calculating a post-hemodialysis extracellular fluid volume based on the difference between pre-hemodialysis and post-hemodialysis uric acid quantities in the extracellular compartment 50 will be described further in detail. A amount of uric acid removed from the extracellular compartment 50 by hemodialysis (which may be referred to as "removal amount of uric acid", hereinbelow) is equal to a difference between an amount of uric acid distributed in the extracellular compartment 50 before hemodialysis and an amount of uric acid distributed in the extracellular compartment 50 after hemodialysis. Here, an amount of urea distributed in the extracellular compartment 50 can be calculated by multiplying an extracellular fluid volume by a uric acid concentration in the extracellular compartment 50. Thus, the formula of Mathematical 1 below holds up. Here, $_{acid}E$ represents removal amount of uric acid, $_{ecf}Vs$ represents pre-hemodialysis extracellular fluid volume, $_{ecr}Ve$ represents post-hemodialysis extracellular fluid volume, $_{acid}Cs$ represents pre-hemodialysis uric acid concentration in the extracellular compartment 50, and $_{acid}Ce$ represents post-hemodialysis uric acid concentration in the extracellular compartment 50. Since uric acid passes through the capillary membrane 56, a uric acid concentration in the intravascular compartment 54 is equal to a uric acid concentration in the interstitial compartment 52. Further, since the extracellular compartment 50 is the combination of the intravascular compartment 54 and the interstitial compartment 52, the uric acid concentration in the extracellular compartment 50 can be considered as the uric acid concentration in the intravascular compartment 54. Thus, the uric acid concentration in the extracellular compartment 50 can be considered as a plasma uric acid concentration.

[Mathematical 1]

$$_{acid}E = {}_{ecf}Vs \times {}_{acid}Cs - {}_{ecf}Ve \times {}_{acid}Ce$$

Next, a difference in water volume in extracellular compartment 50 during hemodialysis will be described. A difference between pre-hemodialysis and post-hemodialysis extracellular fluid volumes is equal to a water volume transferred from the inside of the extracellular compartment 50 to the outside thereof. Further, the water volume transferred from the inside of the extracellular compartment 50 to the outside thereof is equal to a sum of a water volume removed from the inside of body by hemodialysis (which may be referred to simply as "removal volume of water") and a water volume transferred from the extracellular compartment 50 to the intracellular compartment 40 during hemodialysis (which may be referred to as "water volume transferred from the extracellular compartment 50 to the intracellular compartment 40"). Thus, the formula of Mathematical 2 below holds up. Here, $_{dial}EW$ represents removal volume of water and $_{cell}EW$ represents water volume transferred from the extracellular compartment 50 to the intracellular compartment 40.

[Mathematical 2]

$$_{ecf}Vs - _{ecf}Ve = _{dial}EW + _{cell}EW$$

The formula of Mathematical 3 below can be obtained from the above formulas of Mathematical 1 and 2.

[Mathematical 3]

$$_{ecf}Ve = \frac{_{acid}E - (_{dial}EW + _{cell}EW) \times _{acid}Cs}{_{acid}Cs - _{acid}Ce}$$

As described, the pre-hemodialysis uric acid concentration $_{acid}Cs$ in the extracellular compartment 50 is equal to the pre-hemodialysis plasma uric acid concentration, and the post-hemodialysis uric acid concentration $_{acid}Ce$ in the extracellular compartment 50 is equal to the post-hemodialysis plasma uric acid concentration. Further, the pre-hemodialysis uric acid concentration $_{acid}Cs$ and the post-hemodialysis uric acid concentration $_{acid}Ce$ can be acquired as measured values. Furthermore, the removal volume of water $_{dial}EW$ can also be acquired as a measured value. Thus, post-hemodialysis extracellular fluid volume $_{ecf}Ve$ can be calculated using the above formula of Mathematical 3 by acquiring or calculating the water volume $_{cell}EW$ transferred from the extracellular compartment 50 to the intracellular compartment 40 and removal amount of uric acid $_{acid}E$.

The water volume $_{cell}EW$ transferred from the extracellular compartment 50 to the intracellular compartment 40 can be calculated based on change in a sodium concentration in the extracellular compartment 50. Sodium substantially does not pass through the cell membrane 42, while water passes through the cell membrane 42 (see FIG. 2). Further, an extracellular-intracellular sodium concentration ratio is maintained constant. When a sodium concentration in the extracellular compartment 50 changes, water transfers through the cell membrane 42 from the extracellular compartment 50 to the intracellular compartment 40 or from the intracellular compartment 40 to the extracellular compartment 50, such that the extracellular-intracellular sodium concentration ratio does not change with the sodium concentration change in the extracellular compartment 50. When water transfers from the extracellular compartment 50 to the intracellular compartment 40 due to the sodium concentration change in the extracellular compartment 50, sodium in the intracellular compartment 40 is attenuated. On the contrary, when water transfers from the intracellular compartment 40 to the extracellular compartment 50 due to the sodium concentration change in the extracellular compartment 50, sodium in the intracellular compartment 40 is concentrated.

Since the extracellular-intracellular sodium concentration ratio is maintained constant all the time, the formulas of Mathematical 4 and 5 below hold up. Here, $_{na}R$ represents extracellular-intracellular sodium concentration ratio, $_{(icf)na}Cs$ represents pre-hemodialysis sodium concentration in the intracellular compartment 40, $_{(cef)na}Cs$ represents pre-hemodialysis sodium concentration in the extracellular compartment 50, $_{(icf)na}Ce$ represents post-hemodialysis sodium concentration in the intracellular compartment 40, and $_{(ecf)na}Ce$ represents post-hemodialysis sodium concentration in the extracellular compartment 50. Since sodium passes through the capillary membrane 56, the sodium concentration in the extracellular compartment 50 is equal to a serum sodium concentration.

[Mathematical 4]

$$_{na}R = \frac{_{(icf)na}Cs}{_{(ecf)na}Cs}$$

7

[Mathematical 5]

$$_{na}R = \frac{_{(iec)na}C_e}{_{(ec)na}C_e}$$

Since sodium substantially does not pass through the cell membrane 42, the amount of sodium distributed in the intracellular compartment 40 does not change due to hemodialysis. Thus, the formula of Mathematical 6 below holds up. Here, $_{icf}V_s$ represents pre-hemodialysis intracellular fluid volume and $_{icf}V_e$ represents post-hemodialysis intracellular fluid volume.

[Mathematical 6]

$$_{icf}V_s \times {}_{(ic)na}C_s = {}_{icf}V_e \times {}_{(ic)na}C_e$$

The formula of Mathematical 7 below can be obtained from the formulas of Mathematical 4 to 6.

[Mathematical 7]

$$_{icf}V_s \times {}_{(ec)na}C_s = {}_{icf}V_e \times {}_{(ec)na}C_e$$

The water volume $_{cell}EW$ transferred to the intracellular compartment is equal to an increase in the intracellular fluid volume due to hemodialysis. Thus, the formula of Mathematical 8 below holds up.

[Mathematical 8]

$$_{cell}EW = {}_{icf}V_e - {}_{icf}V_s$$

The formula of Mathematical 9 below can be obtained from the formulas of Mathematical 7 and 8.

[Mathematical 9]

$$_{cell}EW = (1 - \frac{_{(ec)na}C_e}{_{(ec)na}C_s}) \times {}_{icf}V_e$$

A post-hemodialysis total body fluid volume is equal to a sum of the post-hemodialysis intracellular fluid volume $_{icf}V_e$ and the post-hemodialysis extracellular fluid volume $_{ecf}V_e$. Thus, the formula of Mathematical 10 below holds up. Here, $_{wb}V_e$ represents post-hemodialysis total body fluid volume.

[Mathematical 10]

$$_{wb}V_e = {}_{icf}V_e + {}_{ecf}V_e$$

The formula of Mathematical 11 below can be obtained from the formulas of Mathematical 9 and 10.

[Mathematical 11]

$$_{cell}EW = (1 - \frac{_{(ecf)na}Ce}{_{(ecf)na}Cs}) \times (_{wb}Ve - _{ecf}Ve)$$

By substituting Mathematical 11 into the formula of Mathematical 3, the formula of Mathematical 3 is rearranged as the formula of Mathematical 12 below.

[Mathematical 12]

$$_{ecf}Ve = \frac{\{(1 - \frac{_{(ecf)na}Ce}{_{(ecf)na}Cs}) \times _{wb}Ve + _{dial}EW\} \times _{acid}Cs - _{acid}E}{(1 - \frac{_{(ecf)na}Ce}{_{(ecf)na}Cs}) \times _{acid}Cs - (_{acid}Cs - _{acid}Ce)}$$

As described, the pre-hemodialysis sodium concentration $_{(ecf)na}Cs$ in the extracellular compartment 50 is equal to the pre-hemodialysis serum sodium concentration, and the post-hemodialysis sodium concentration $_{(ecf)na}Ce$ in the extracellular compartment 50 is equal to the post-hemodialysis serum sodium concentration. Thus, the post-hemodialysis sodium concentration $_{(ecf)na}Ce$ in the extracellular compartment 50 and the pre-hemodialysis sodium concentration $_{(ecf)na}Cs$ in the extracellular compartment 50 can be acquired as measured values. Further, as described, the removal volume of water $_{dial}EW$, the pre-hemodialysis uric acid concentration $_{acid}Cs$ in the extracellular compartment 50, and the post-hemodialysis uric acid concentration $_{acid}Ce$ in the extracellular compartment 50 can also be acquired as measured values. Thus, the post-hemodialysis extracellular fluid volume $_{ecf}Ve$ can be calculated using the formula of Mathematical 12 by acquiring or calculating the post-hemodialysis total body fluid volume $_{wb}Ve$ and the removal amount of uric acid $_{acid}E$. Methods for calculating the post-hemodialysis total body fluid volume $_{wb}Ve$ and the removal amount of uric acid $_{acid}E$ will be described hereinbelow.

First, a method for calculating the post-hemodialysis total body fluid volume $_{wb}Ve$ will be described. Since urea passes through the cell membrane 24 (see FIG. 2), it is distributed over the entire fluid compartment in the body. Thus, the post-hemodialysis total body fluid volume $_{wb}Ve$ is calculated focusing on the urea distribution.

An amount of urea removed by hemodialysis (which may be referred to as "amount of urea", hereinbelow) is equal to the difference between a pre-hemodialysis amount of urea distributed in the fluid compartment and a post-hemodialysis amount of urea distributed in the fluid compartment. Further, a pre-hemodialysis total body fluid volume is equal to a sum of the post-hemodialysis total body fluid volume $_{wb}Ve$ and the removal volume of water $_{dial}EW$. Thus, the formula of Mathematical 13 below holds up. Here, $_{urea}E$ represents removal amount of urea, $_{urea}Cs$ represents pre-hemodialysis urea concentration in the fluid compartment, and $_{urea}Ce$ represents post-hemodialysis urea concentration in the fluid compartment.

[Mathematical 13]

$$_{urea}E = (_{wb}Ve + _{dial}EW) \times _{urea}Cs - _{wb}Ve \times _{urea}Ce$$

The formula of Mathematical 14 below is obtained by rearranging the above formula of Mathematical 13.

[Mathematical 14]

$$_{wb}Ve = \frac{_{urea}E - _{dial}EW \times _{urea}Cs}{_{urea}Cs - _{urea}Ce}$$

The pre-hemodialysis urea concentration $_{urea}Cs$ in the fluid compartment is equal to the pre-hemodialysis plasma urea concentration, and the post-hemodialysis urea concentration $_{urea}Ce$ in the fluid compartment is equal to the post-hemodialysis plasma urea concentration. Thus, the pre-hemodialysis urea concentration $_{urea}Cs$ in the fluid compartment and the post-hemodialysis urea concentration $_{urea}Ce$ in the fluid compartment can be acquired as measured values. Further, as described, the removal volume of water $_{dial}EW$ can also be acquired as a measured value. Thus, the post-hemodialysis total body fluid volume $_{wb}Ve$ can be calculated by acquiring or calculating the removal amount $_{urea}E$ of urea.

The removal amount $_{urea}E$ of urea may, for example, be acquired by measuring the amount of urea removed into the dialysate or be calculated based on a dialyzer clearance for urea used in hemodialysis. A method for calculating the removal amount $_{urea}E$ of urea from the dialyzer clearance for urea will be described hereinbelow.

It is known that the plasma urea concentration decreases exponentially during hemodialysis. Thus, the formula of Mathematical 15 below holds up. Here, $_{urea}C(t)$ represents plasma urea concentration at a time t during hemodialysis, and $_{urea}A$ represents a coefficient calculated using the formula of Mathematical 16 below. Td represents hemodialysis period.

[Mathematical 15]

$$_{urea}C(t) = _{urea}Cs \times \exp(_{urea}A \times t)$$

[Mathematical 16]

$$_{urea}A = \frac{\ln(_{urea}Ce/_{urea}Cs)}{Td}$$

The formula of Mathematical 17 below can be obtained from the formulas of Mathematical 15 and 16.

[Mathematical 17]

$$_{urea}C(t) = _{urea}Cs \times (_{urea}Ce/_{urea}Cs)^{\wedge}(\frac{t}{Td})$$

A urea removal rate at the time t during hemodialysis can be calculated by multiplying the dialyzer clearance for urea by the plasma urea concentration $_{urea}C(t)$ at the time t. Thus, the formula of Mathematical 18 below holds up. Here, $_{urea}F(t)$ represents urea removal rate at the time t during hemodialysis and $_{urea}K$ represents the dialyzer clearance for urea.

[Mathematical 18]

$$_{urea}F(t) = _{urea}K \times _{urea}C(t)$$

It is known that the dialyzer clearance for urea can be calculated, using a known formula, from the dialyzer overall mass transfer-area coefficient for urea described in the catalogue accompanying the dialyzer (which may be referred to as "catalogue value of the dialyzer overall mass transfer-area coefficient for urea", hereinbelow), a blood flow rate passing through the dialyzer during hemodialysis, and a dialysate flow rate passing through the dialyzer during hemodialysis. Urea is distributed in both plasma and blood cells. That is, urea is distributed throughout blood. Thus, the blood flow rate passing through the dialyzer is used to calculate the dialyzer clearance for urea. Since the blood flow rate passing through the dialyzer is substantially constant during hemodialysis, the dialyzer clearance $_{urea}K$ for urea is a constant independent of the time t. Thus, the removal amount $_{urea}E$ of urea can be calculated by integrating the above formula of Mathematical 18 from t=0 to t=Td. Accordingly, the removal amount $_{urea}E$ of urea is calculated using the formula of Mathematical 19 below.

[Mathematical 19]

$$_{urea}E = \int_{0}^{Td} \{_{urea}K \times {}_{urea}C(t)\} dt$$

The formula of Mathematical 20 below can be obtained from the formulas of Mathematical 17 and 19.

[Mathematical 20]

$$_{urea}E = {}_{urea}K \times {}_{urea}Cs \times \frac{Td}{\ln({}_{urea}Ce/{}_{urea}Cs)} \{({}_{urea}Ce/{}_{urea}Cs) - 1\}$$

The hemodialysis period Td can be acquired as a measured value. Further, as described, the pre-hemodialysis plasma urea concentration $_{urea}Cs$ and the post-hemodialysis plasma urea concentration $_{urea}Ce$ can be acquired as measured values.

Accordingly, the removal amount $_{urea}E$ of urea can be calculated by substituting the dialyzer clearance $_{urea}K$ for urea, which is calculated from the catalogue value of dialyzer overall mass transfer-area coefficient for urea, the blood flow rate passing through the dialyzer during hemodialysis, and the dialysate flow rate passing through the dialyzer during hemodialysis, and the acquired hemodialysis period Td, pre-hemodialysis plasma urea concentration $_{urea}Cs$, and post-hemodialysis plasma urea concentration $_{urea}Ce$ into the formula of Mathematical 20. Then, the post-hemodialysis total body fluid volume $_{wb}Ve$ can be calculated by substituting the calculated removal amount $_{urea}E$ of urea and the acquired pre-hemodialysis plasma urea concentration $_{urea}Cs$, post-hemodialysis plasma urea concentration $_{urea}Ce$, and removal volume of water $_{dial}EW$ into the above formula of Mathematical 14. That is, the post-hemodialysis total body fluid volume $_{wb}Ve$ can be calculated from the pre-hemodialysis plasma urea concentration $_{urea}Cs$, the post-hemodialysis plasma urea concentration $_{urea}Ce$, the removal volume of water $_{dial}EW$, the hemodialysis period Td, and the dialyzer clearance $_{urea}K$ for urea.

In the present embodiment, the dialyzer clearance $_{urea}K$ for urea is calculated using the catalogue value of dialyzer overall mass transfer-area coefficient for urea, however, it is not limited thereto. For example, the catalogue of the dialyzer may describe a dialyzer clearance for urea at a specific blood flow rate and a specific dialysate flow rate, instead of the dialyzer overall mass transfer-area coefficient for urea. In this case, the dialyzer overall mass transfer-area coefficient for urea may be calculated from the blood flow rate, the dialysate flow rate, and the dialyzer clearance for urea described in the catalogue, and then the dialyzer clearance for urea may be calculated from the calculated dialyzer overall mass transfer-area coefficient for urea, the blood flow rate passing through the dialyzer during hemodialysis, and the dialysate flow rate passing through the dialyzer during hemodialysis.

Next, the removal amount of uric acid $_{acid}E$ will be described. The removal amount of uric acid $_{acid}E$ may, for example, be acquired by measuring the amount of uric acid removed into the dialysate or be calculated based on the plasma uric acid concentration and the dialyzer clearance for uric acid used in hemodialysis. Hereinbelow, a method for calculating the removal amount of uric acid $_{acid}E$ from the plasma uric acid concentration and the dialyzer clearance for uric acid used in hemodialysis will be described.

It is known that the plasma uric acid concentration decreases exponentially during hemodialysis. Thus, the formula of Mathematical 21 below holds up. Here, $_{acid}C(t)$ represents plasma uric acid concentration at the time t during hemodialysis and $_{acid}A$ represents a coefficient calculated using the formula of Mathematical 22.

[Mathematical 21]

$$_{acid}C(t) = {}_{acid}Cs \times \exp({}_{acid}A \times t)$$

[Mathematical 22]

$$_{acid}A = \frac{\ln({}_{acid}Ce/{}_{acid}Cs)}{Td}$$

The formula of Mathematical 23 below can be obtained from the formulas of Mathematical 21 and 22.

[Mathematical 23]

$$_{acid}C(t) = {}_{acid}Cs \times ({}_{acid}Ce/{}_{acid}Cs)^{\wedge}(\frac{t}{Td})$$

The uric acid removal rate at the time t during hemodialysis can be calculated by multiplying the dialyzer clearance for uric acid at the time t by the plasma uric acid concentration $_{acid}C(t)$ at the time t. Thus, the formula of Mathematical 24 below holds up. Here, $_{acid}F(t)$ represents uric acid removal rate at the time t during hemodialysis and $_{acid}K(t)$ represents dialyzer clearance for uric acid at the time t during hemodialysis.

[Mathematical 24]

$$_{acid}F(t) = {}_{acid}K(t) \times {}_{acid}C(t)$$

The dialyzer clearance $_{urea}K$ for urea is a constant, while the dialyzer clearance $_{acid}K(t)$ for uric acid is a variable depending on the time t. Urea is distributed in both plasma and blood cells in the intravascular compartment 54 and passes through red blood cell membranes, which are cell membranes. On the other hand, although uric acid is distributed in both plasma and blood cells in the intravascular compartment 54 similar to urea (Nagendra S, et al: A comparative study of plasma uric acid, erythrocyte uric acid and urine uric acid levels in type 2 diabetic subjects. Merit Research Journal 3: 571-574, 2015), it does not pass through red blood cell membranes, which are cell membranes. Thus, uric acid is removed only from a plasma compartment during hemodialysis (Eric Descombes, et al: Diffusion kinetics of urea, creatinine and uric acid in blood during hemodialysis. Clinical implications. Clinical Nephrology 40: 286-295, 1993). Accordingly, not the blood flow rate passing through the dialyzer but a plasma flow rate passing through the dialyzer is used to calculate the dialyzer clearance $_{acid}K(t)$ for uric acid. By the way, as the blood is concentrated by the water removal during hemodialysis, the hematocrit value increases over time during hemodialysis. Thus, the plasma flow rate is not constant during hemodialysis. Accordingly, the dialyzer clearance $_{acid}K(t)$ for uric acid, which is calculated using the plasma flow rate passing through the dialyzer, also changes with the change in the plasma flow rate passing through the dialyzer over time. That is, the dialyzer clearance $_{acid}K(t)$ for uric acid is a variable depending on the time t.

Since the dialyzer clearance $_{urea}K$ for uric acid is a constant, the removal amount $_{urea}E$ of urea is calculated by integrating the formula of Mathematical 18 from t=0 to t=Td, as shown in Mathematical 19. On the contrary, the dialyzer clearance $_{acid}K(t)$ for uric acidis a variable depending on the time t, thus the removal amount of uric acid $_{acid}E$ is calculated by adding up the uric acid removal rate at the time t calculated using the formula of Mathematical 24 while changing the time t from t=0 up to t=Td at regular intervals (e.g., at intervals of 0.1 min.). That is, the removal amount of uric acid $_{acid}E$ is calculated using the formula of Mathematical 25 below.

[Mathematical 25]

$$_{acid}E = \sum_{t=0}^{Td} \{_{acid}K(t) \times _{acid}C(t)\}$$

The hemodialysis period Td can be acquired as a measured value. Further, the plasma uric acid concentration $_{acid}C(t)$ at the time t can be calculated using the above formula of Mathematical 23. Thus, the removal amount of uric acid $_{acid}E$ can be calculated using the formula of Mathematical 25 by acquiring or calculating the dialyzer clearance $_{acid}K(t)$ for uric acid at the time t.

The dialyzer clearance $_{acid}K(t)$ for uric acid at the time t can be calculated using a known formula for calculating a dialyzer clearance for solute. That is, the dialyzer clearance $_{acid}K(t)$ for uric acid at the time t can be calculated using the formula of Mathematical 26 below. Here, $_{acid}KoA$ represents dialyzer overall mass transfer-area coefficient for uric acid, $Q_{Pt}$ represents plasma flow rate passing through the dialyzer at the time t during hemodialysis, and $Q_D$ represents dialysate flow rate passing through the dialyzer.

[Mathematical 26]

$$_{acid}K(t) = \frac{1 - exp\{_{acid}KoA(\frac{1}{Q_{Pt}} - \frac{1}{Q_D})\}}{\frac{1}{Q_D} - \frac{1}{Q_{Pt}} exp\{_{acid}KoA(\frac{1}{Q_{Pt}} - \frac{1}{Q_D})\}}$$

The dialysate flow rate $Q_D$ passing through the dialyzer can be acquired as a measured value. Thus, the dialyzer clearance $_{acid}K(t)$ for uric acid at the time t can be calculated by acquiring or calculating the dialyzer overall mass transfer-area coefficient $_{acid}KoA$ for uric acid and the plasma flow rate $Q_{Pt}$ passing through the dialyzer at the time t.

Methods for calculating the dialyzer overall mass transfer-area coefficient $_{acid}KoA$ for uric acid and the plasma flow rate $Q_{Pt}$ passing through the dialyzer at the time t, which are used to calculate the dialyzer clearance $_{acid}K(t)$ for uric acid at the time t during hemodialysis, will be described. First, a method for calculating the dialyzer overall mass transfer-area coefficient $_{acid}KoA$ for uric acid will be described.

Solute overall mass transfer-area coefficients are proportional to diffusion coefficients. Thus, the formula of Mathematical 27 below holds up among the dialyzer overall mass transfer-area coefficient for urea, urea diffusion coefficient, dialyzer overall mass transfer-area coefficient for uric acid, and uric acid diffusion coefficient. Here, $_{acid}D$ represents uric acid diffusion coefficient, $_{urea}D$ represents urea diffusion coefficient, and $_{urea}KoA$ represents dialyzer overall mass transfer-area coefficient for urea.

[Mathematical 27]

$$_{acid}KoA = \frac{_{acid}D}{_{urea}D} \times _{urea}KoA$$

It is known that the uric acid diffusion coefficient $_{acid}D$ is $1.4 \times 10^6$ cm$^2$/sec and the urea diffusion coefficient $_{urea}D$ is $2.6 \times 10^6$ cm$^2$/sec. The formula of Mathematical 28 below is obtained by substituting these numerical values into the formula of Mathematical 27.

[Mathematical 28]

$$_{acid}KoA = 0.53846\ _{urea}KoA$$

The dialyzer overall mass transfer-area coefficient $_{urea}KoA$ for urea is described in the catalogue of the dialyzer. Thus, the dialyzer overall mass transfer-area coefficient $_{acid}KoA$ for uric acid can be calculated by substituting the catalogue value of the dialyzer overall mass transfer-area coefficient for urea as the dialyzer overall mass transfer-area coefficient $_{urea}KoA$ for urea in the formula of Mathematical 28.

Next, a method for calculating the plasma flow rate $Q_{Pt}$ through the dialyzer at the time t will be described. Plasma means a part of blood from which blood cell components are excluded. Thus, the plasma volume in blood can be expressed as shown in Mathematical 29 below. Here, PV represents plasma volume, BV represents blood volume, and Ht represents hematocrit value.

[Mathematical 29]

$$PV = \{1 - Ht/100\} \times BV$$

The formula of Mathematical 30 is obtained by rearranging the formula of Mathematical 29 into a relationship between the blood flow rate and the plasma flow rate passing through the dialyzer at the time t. Here, Ht(t) represents hematocrit value (%) at the time t during hemodialysis, $Q_B$ represents blood flow rate passing through the dialyzer at the time t during hemodialysis, and $Q_{Pt}$ represents plasma flow rate passing through the dialyzer at the time t during hemodialysis. The blood flow rate $Q_B$ passing through the dialyzer is constant during hemodialysis.

[Mathematical 30]

$$Q_{Pt} = \{1 - Ht(t)/100\} \times Q_B$$

The blood flow rate $Q_B$ passing through the dialyzer does not change over time and can be acquired as a measured value. Thus, plasma flow rate $Q_{Pt}$ passing through the dialyzer can be calculated by calculating the hematocrit value Ht(t) at the time t. Hereinbelow, a method for calculating the hematocrit value Ht(t) at the time t will be described.

With constant speed of water removal, the blood volume in the body decreases substantially in a linear fashion during hemodialysis. This has been confirmed by the inventors studying measurement result from a BV meter (blood volume meter). Thus, a blood volume BV(t) at the time t during hemodialysis is expressed as in the formula of Mathematical 31, where BVs represents blood volume at t=0. Here, $\alpha$ is usually a negative value.

[Mathematical 31]

$$BV(t) = \alpha \times t + BVs$$

In Mathematical 32, the value $\alpha$ can be calculated by substituting t=Td in the formula of Mathematical 31, where BVe represents a blood volume at the end of hemodialysis.

[Mathematical 32]

$$BVe = \alpha \times Td + BVs$$

The formula of Mathematical 33 below is obtained by rearranging the above formula of Mathematical 32.

[Mathematical 33]

$$\alpha = \frac{BVe - BVs}{Td}$$

The formula of Mathematical 34 below is obtained by substituting the $\alpha$ calculated in the formula of Mathematical 33 into the above formula of Mathematical 31.

[Mathematical 34]

$$BV(t) = \frac{BVe - BVs}{Td} \times t + BVs$$

The total number of red blood cells in the body is constant and does not change over time. This means that the total red blood cell volume is also constant and does not change over time. The total red blood cell volume in the body is calculated by multiplying the blood volume in the body by one-hundredth of the hematocrit value. Thus, the formulas of Mathematical 35 to 37 are obtained. TE represents total red blood cell volume in the body.

[Mathematical 35]

$$BV(t) \times Ht(t)/100 = TE$$

[Mathematical 36]

$$BVs \times Hts/100 = TE$$

[Mathematical 37]

$$BVe \times Hte/100 = TE$$

The formula of Mathematical 38 below is obtained from the above formulas of Mathematical 34 to 37.

[Mathematical 38]

$$Ht(t) = \frac{Td}{(Hts/Hte - 1) \times t + Td} \times Hts$$

A pre-hemodialysis hematocrit value Hts and a post-hemodialysis hematocrit value Hte can be acquired as measured values. Further, as described, the hemodialysis period Td can be acquired as a measured value. Thus, the hematocrit value Ht(t) at the time t during hemodialysis can be calculated by substituting the pre-hemodialysis hematocrit value Hts, the post-hemodialysis hematocrit value Hte, and the hemodialysis period Td into the formula of Mathematical 38. Then, the plasma flow rate $Q_{Pt}$ passing through the dialyzer at the time t can be calculated by substituting the hematocrit value Ht(t) at the time t calculated in the formula of Mathematical 38 and the acquired blood flow rate $Q_B$ passing through the dialyzer into the formula of Mathematical 30. That is, the plasma flow rate $Q_{Pt}$ passing through the dialyzer at the time t can be calculated from the pre-hemodialysis hematocrit value Hts, the post-hemodialysis hematocrit value Hte, the hemodialysis period Td, and the blood flow rate $Q_B$.

The dialyzer clearance $_{acid}K(t)$ for uric acid at the time t can be calculated by substituting the dialyzer overall mass transfer-area coefficient $_{acid}KoA$ for uric acid calculated using the formula of Mathematical 28, the plasma flow rate $Q_{Pt}$ passing through the dialyzer at the time t calculated using the formulas of Mathematical 30 and 38, and the acquired dialysate flow rate $Q_D$ passing through the dialyzer into the formula of Mathematical 26. That is, the dialyzer clearance $_{acid}K(t)$ for uric acid at the time t can be calculated from the pre-hemodialysis hematocrit value Hts, the post-hemodialysis hematocrit value Hte, the hemodialysis period Td, the blood flow rate $Q_B$ passing through the dialyzer, the dialysate flow rate $Q_D$ passing through the dialyzer, and the catalogue value of the dialyzer overall mass transfer-area coefficient $_{urea}K_OA$ for urea.

Further, the removal amount of uric acid $_{acid}E$ can be calculated by substituting the dialyzer clearance $_{acid}K(t)$ for uric acid at the time t calculated using the formula of mathematical 26 and the plasma uric acid concentration $_{acid}C(t)$ at the time t calculated using the formula of Mathematical 23 into the formula of Mathematical 25. That is, the removal amount of uric acid $_{acid}E$ can be calculated from the pre-hemodialysis plasma uric acid concentration $_{acid}Cs$, the post-hemodialysis plasma uric acid concentration $_{acid}Ce$, the pre-hemodialysis hematocrit value Hts, the post-hemodialysis hematocrit value Hte, the hemodialysis period Td, the blood flow rate $Q_B$ passing through the dialyzer, the dialysate flow rate $Q_D$ passing through the dialyzer, and the catalogue value of the dialyzer overall mass transfer-area coefficient $_{urea}K_OA$ for urea.

Further, the post-hemodialysis extracellular fluid volume $_{ecf}Ve$ can be calculated by substituting the post-hemodialysis total body fluid volume $_{wb}Ve$ calculated using the formula of Mathematical 14, the removal amount of uric acid $_{acid}E$ calculated using the formula of Mathematical 25, and the acquired pre-hemodialysis plasma uric acid concentration $_{acid}Cs$, post-hemodialysis plasma uric acid concentration $_{acid}Ce$, pre-hemodialysis serum sodium concentration $_{(ecf)na}Cs$, post-hemodialysis serum sodium concentration $_{(ecf)na}Ce$, and removal volume of water $_{dial}EW$ into the formula of Mathematical 12.

As described, in the present embodiment, the post-hemodialysis extracellular fluid volume $_{ecf}Ve$ can be calculated from the pre-hemodialysis plasma uric acid concentration $_{acid}Cs$, the post-hemodialysis plasma uric acid concentration $_{acid}Ce$, the pre-hemodialysis plasma urea concentration $_{urea}Cs$, the post-hemodialysis plasma urea concentration $_{urea}Ce$, the pre-hemodialysis serum sodium concentration $_{(ecf)na}Cs$, the post-hemodialysis serum sodium concentration $_{(ecf)na}Ce$, the pre-hemodialysis hematocrit value Hts, the post-hemodialysis hematocrit value Hte, the removal volume of water $_{dial}EW$, the hemodialysis period Td, the blood flow rate $Q_B$ passing through the dialyzer, the dialysate flow rate $Q_D$ passing through the dialyzer, and the catalogue value of the dialyzer overall mass transfer-area coefficient $_{urea}KoA$ for urea.

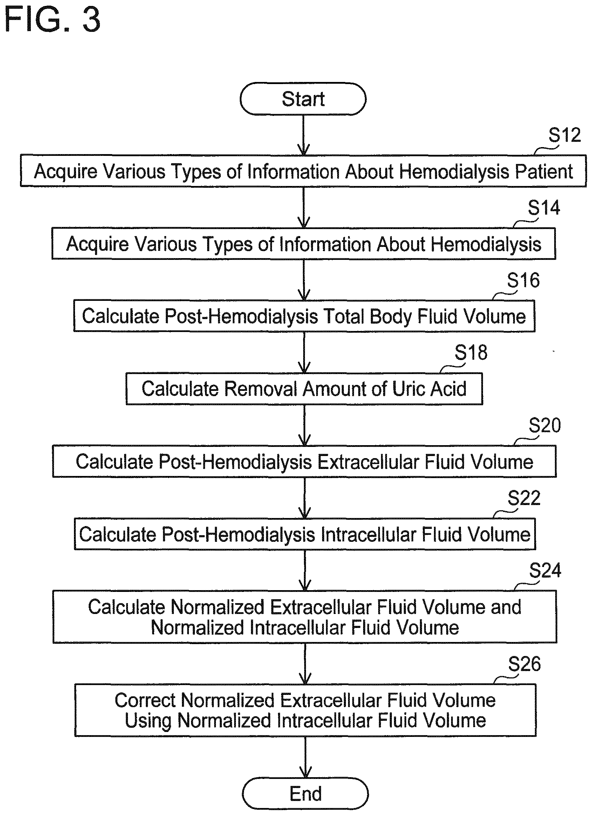

Next, a process of calculating the post-hemodialysis extracellular fluid volume $_{ecf}Ve$ by the extracellular fluid volume calculator 10 will be described. FIG. 3 shows a flowchart of an exemplary process of calculating the post-hemodialysis extracellular fluid volume $_{ecf}Ve$ by the extracellular fluid volume calculator 10. In the process of FIG. 3, steps S12 to S20 are for calculating the post-hemodialysis extracellular fluid volume $_{ecf}Ve$, step S22 is for calculating the post-hemodialysis intracellular fluid volume $_{icf}Ve$, step S24 is for standardizing the post-hemodialysis extracellular fluid volume $_{ecf}Ve$ and the post-hemodialysis intracellular fluid volume $_{icf}Ve$ with ideal body weight, and step S26 is for correcting the normalized extracellular fluid volume with the normalized intracellular fluid volume to calculate a corrected post-hemodialysis normalized extracellular fluid volume (corrected nECVe).

As shown in FIG. 3, the processor 12 firstly acquires various types of information about the hemodialysis patient (S12). The various types of information about the hemodialysis patient include, for example, the pre-hemodialysis and post-hemodialysis plasma uric acid concentrations $_{acid}Cs$ and $_{acid}Ce$, the pre-hemodialysis and post-hemodialysis plasma urea concentrations $_{urea}Cs$ and $_{urea}Ce$, the pre-hemodialysis and post-hemodialysis serum sodium concentrations $_{(ecf)na}Cs$ and $_{(ecf)na}Ce$, the pre-hemodialysis and post-hemodialysis hematocrit values Hts and Hte, the body height of the hemodialysis patient, and the like. The various types of information about the hemodialysis patient are acquired as described below, for example. How the pre-hemodialysis and post-hemodialysis plasma uric acid concentrations $_{acid}Cs$ and $_{acid}Ce$ are acquired will be described as an example. Firstly, blood samples are collected from the hemodialysis patient before and after hemodialysis. Then, the blood sample collected before hemodialysis is subjected to centrifugal process to separate the blood into blood cells and plasma, and the uric acid concentration $_{acid}Cs$ in the separated plasma is measured, and further, the blood sample collected after hemodialysis is subjected to centrifugal process to separate the blood into blood cells and plasma, and the uric acid concentration $_{acid}Ce$ in the separated plasma is measured. How the uric acid concentrations are measured is not particularly limited. The operator inputs the measured pre-hemodialysis and post-hemodialysis plasma uric acid concentrations $_{acid}Cs$ and $_{acid}Ce$ into the interface 30. The inputted pre-hemodialysis and post-hemodialysis plasma uric acid concentrations $_{acid}Cs$ and $_{acid}Ce$ are outputted from the interface 30 to the processor 12 and stored in the patient's information storage unit 14. The pre-hemodialysis and post-hemodialysis plasma urea concentrations $_{urea}Cs$ and $_{urea}Ce$ and the pre-hemodialysis and post-hemodialysis serum sodium concentrations $_{(ecf)na}Cs$ and $_{(ecf)na}Ce$ are similarly measured. Further, the pre-hemodialysis and post-hemodialysis hematocrit values Hts and Hte are respectively measured from the blood samples collected from the hemodialysis patient before and after hemodialysis. These measured values are stored in the patient's information storage unit 14 through the interface 30. The body height of the hemodialysis patient is also stored in the patient's information storage unit 14 through the interface 30.

Next, the processor 12 acquires various types of information about hemodialysis (S14). The various types of information about hemodialysis include, for example, the removal volume of water $_{dial}EW$, the hemodialysis period Td, the blood flow rate $Q_B$ passing through the dialyzer, the dialysate flow rate $Q_D$ passing through the dialyzer, the catalogue value of dialyzer overall mass transfer-area coefficient $_{urea}KoA$ for urea, and the like. The removal volume of water $_{dial}EW$, the hemodialysis period Td, the blood flow rate $Q_B$ passing through the dialyzer, the dialysate flow rate $Q_D$ passing through the dialyzer, and the catalogue value of dialyzer overall mass transfer-area coefficient $_{urea}KoA$ for urea are inputted to the interface 30 by the operator. Then, the removal volume of water $_{dial}EW$, the hemodialysis period Td, the blood flow rate $Q_B$ passing through the dialyzer, the dialysate flow rate $Q_D$ passing through the dialyzer, and the catalogue value of dialyzer overall mass transfer-area coefficient $_{urea}KoA$ for urea are outputted from the interface 30 to the processor 12 and stored in the hemodialysis information storage unit 16.

In the present embodiment, step S14 is carried out after step S12, however, no limitations are placed on the order of these steps. For example, step S14 may be carried out before step S12. Further, the acquisition order for the plural pieces of information acquired in step S12 and the plural pieces of information acquired in step S14 is not particularly limited. Any acquisition order may be applied as long as all of the items of step S12 and step S14 can be acquired. For example, the information to be acquired in step S14 may be acquired before all of the plural pieces of information to be acquired in step S12 are acquired.

Next, the calculation unit 20 calculates the post-hemodialysis total body fluid volume $_{wb}Ve$ using the information about the hemodialysis patient acquired in step S12 and the information about hemodialysis acquired in step S14 (S16). The post-hemodialysis total body fluid volume $_{wb}Ve$ is calculated using the formulas of Mathematical 14 and 20 stored in the calculation method storage unit 18. Specifically, the calculation unit 20 firstly substitutes the pre-hemodialysis and post-hemodialysis plasma urea concentrations $_{urea}Cs$ and $_{urea}Ce$ stored in the patient's information storage unit 14, the hemodialysis period Td stored in the hemodialysis information storage unit 16, and the catalogue value of the dialyzer overall mass transfer-area coefficient $_{urea}KoA$ for urea into the formula of Mathematical 20 to calculate the removal amount $_{urea}E$ of urea. Then, the calculation unit 20 substitutes the removal amount $_{urea}E$ of urea calculated using the formula of Mathematical 20, the pre-hemodialysis and post-hemodialysis plasma urea concentrations $_{urea}Cs$ and $_{urea}Ce$ stored in the patient's information storage unit 14, and the removal volume of water $_{dial}EW$ stored in the hemodialysis information storage unit 16 into the formula of Mathematical 14 to calculate the post-hemodialysis total body fluid volume $_{wb}Ve$. The calculated post-hemodialysis total body fluid volume $_{wb}Ve$ is stored in the patient's information storage unit 14.

In the present embodiment, the removal amount $_{urea}E$ of urea is calculated by the calculation unit 20, however, it is not limited thereto. For example, the removal amount $_{urea}E$ of urea may be acquired by measuring the amount of urea removed into the dialysate. In this case, the operator inputs the removal amount of urea acquired by the measurement into the interface 30 and the removal amount of urea outputted from the interface 30 is stored in the hemodialysis information storage unit 16.

Next, the calculation unit 20 calculates the removal amount of uric acid $_{acid}E$ using the information about the hemodialysis patient acquired in step S12 and the information about hemodialysis acquired in step S14 (S18). The removal amount of uric acid $_{acid}E$ is calculated using the formulas of Mathematical 23 to 26, 28, 30, and 38 stored in the calculation method storage unit 18.

Specifically, the calculation unit 20 firstly substitutes the pre-hemodialysis and post-hemodialysis hematocrit values Hts and Hte stored in the patient's information storage unit 14 and the hemodialysis period Td stored in the hemodialysis information storage unit 16 into the formula of Mathematical 38 to calculate the hematocrit value Ht(t) at the time t. Then, the calculation unit 20 substitutes the hematocrit value Ht(t) at the time t calculated using the formula of Mathematical 38 and the blood flow rate $Q_B$ passing through the dialyzer stored in the hemodialysis information storage unit 16 into the formula of Mathematical 30 to calculate the plasma flow rate $Q_{Pt}$ passing through the dialyzer at the time t. Next, the calculation unit 20 substitutes the catalogue value of the dialyzer overall mass transfer-area coefficient $_{urea}KoA$ for urea stored in the hemodialysis information storage unit 16 into the formula of Mathematical 28 to calculate the dialyzer overall mass transfer-area coefficient $_{acid}KoA$ for uric acid. Further, the calculation unit 20 substitutes the plasma flow rate $Q_{Pt}$ passing through the dialyzer at the time t calculated using the formula of Mathematical 30, the dialysate flow rate $Q_D$ passing through the dialyzer stored in the hemodialysis information storage unit 16, and the dialyzer overall mass transfer-area coefficient $_{acid}KoA$ for uric acid calculated using the formula of Mathematical 28 into the formula of Mathematical 26 to calculate the dialyzer clearance $_{acid}K(t)$ for uric acid at the time t. Then, the calculation unit 20 substitutes the pre-hemodialysis and post-hemodialysis plasma uric acid concentrations $_{acid}Cs$ and $_{acid}Ce$ stored in the patient's information storage unit 14 and the hemodialysis period Td stored in the hemodialysis information storage unit 16 into the formula of Mathematical 23 to calculate the plasma uric acid concentration $_{acid}C(t)$ at the time t. Next, the calculation unit 20 substitutes the plasma uric acid concentration $_{acid}C(t)$ at the time t calculated using the formula of Mathematical 23 and the dialyzer clearance $_{acid}K(t)$ for uric acid at the time t calculated using the formula of Mathematical 26 into the formula of Mathematical 24 to calculate the uric acid removal rate $_{acid}F(t)$ at the time t. Finally, the calculation unit 20 adds up the uric acid removal rate $_{acid}F(t)$ at the time t calculated using the formula of Mathematical 24 while changing the time t from t=0 up to t=Td at regular intervals according to the formula of Mathematical 25 to calculate the removal amount of uric acid $_{acid}E$. The calculated removal amount of uric acid $_{acid}E$ is stored in the hemodialysis information storage unit 16.

In the present embodiment, the removal amount of uric acid $_{acid}E$ is calculated by the calculation unit 20, however, it is not limited thereto. For example, the removal amount of uric acid $_{acid}E$ may be acquired by measuring the amount of uric acid removed into the dialysate. In this case, the operator inputs the removal amount of uric acid acquired by the measurement into the interface 30 and the removal amount of uric acid outputted from the interface 30 is stored in the hemodialysis information storage unit 16. Further, in the present embodiment, step S18 is carried out after step S16, however, no limitations are placed on the order of these steps. For example, step S18 may be carried out before step S16.

Next, the calculation unit 20 calculates the post-hemodialysis extracellular fluid volume $_{ecf}Ve$ (S20). The post-hemodialysis extracellular fluid volume $_{ecf}Ve$ is calculated using the formula of Mathematical 12. Specifically, the calculation unit 20 substitutes the post-hemodialysis total body fluid volume $_{wb}Ve$ calculated in step S16, the removal amount of uric acid $_{acid}E$ calculated in step S18, the pre-hemodialysis and post-hemodialysis plasma uric acid concentrations $_{acid}Cs$ and $_{acid}Ce$ and serum sodium concentrations $_{(ecf)na}Cs$ and $_{(ecf)na}Ce$ stored in the patient's information storage unit 14, and the removal volume of water $_{dial}EW$ stored in the hemodialysis information storage unit 16 into the formula of Mathematical 12 to calculate the post-hemodialysis extracellular fluid volume $_{ecf}Ve$. As above, the post-hemodialysis extracellular fluid volume $_{ecf}Ve$ can be calculated as a specific numerical value from the various numerical values that can be easily acquired from the blood of the hemodialysis patient before and after hemodialysis and the various numerical values about hemodialysis that can be easily acquired.

Next, the calculation unit 20 calculates the post-hemodialysis intracellular fluid volume $_{icf}Ve$ (S22). The post-hemodialysis intracellular fluid volume can be calculated by subtracting the post-hemodialysis extracellular fluid volume $_{ecf}Ve$ from the post-hemodialysis total body fluid volume $_{wb}Ve$. Thus, the formula of Mathematical 39 below holds up. Here, $_{icf}Ve$ represents post-hemodialysis intracellular fluid volume.

$$[\text{Mathematical 39}]$$

$$_{icf}Ve = {}_{wb}Ve - {}_{ecf}Ve$$

The calculation unit 20 substitutes the post-hemodialysis total body fluid volume $_{wb}Ve$ calculated in step S16 and the post-hemodialysis extracellular fluid volume $_{ecf}Ve$ calculated in step S20 into the formula of Mathematical 39 to calculate the post-hemodialysis intracellular fluid volume $_{icf}Ve$.

Next, the calculation unit 20 calculates a post-hemodialysis extracellular fluid volume per kilogram of ideal body weight from the post-hemodialysis extracellular fluid volume $_{ecf}Ve$ calculated in step S20, and also calculates a post-hemodialysis intracellular fluid volume per kilogram of the ideal body weight from the post-hemodialysis intracellular fluid volume $_{icf}Ve$ calculated in step S22 (S24). It is known that a healthy person in standard body shape has an extracellular fluid volume of approximately 200 mL per kilogram of the actual body weight and an intracellular fluid volume of approximately 400 mL per kilogram of the actual body weight. On the contrary, the fat amount of a hemodialysis patient is often different from that of the healthy person in standard body shape, and it is also different among patients. Thus, even when the patients have the same extracellular fluid volume and total body fluid volume, their actual body weights differ from each other if their fat amounts are different. Therefore, even when they have the same extracellular fluid volume and total body fluid volume, their extracellular fluid volume and intracellular fluid volume per kilogram of the actual body weight differ from each other if their actual body weights are different due to the difference in fat amount. That is, even when the extracellular fluid volume and intracellular fluid volume of a patient are the same as those of a healthy person in standard body shape, if they differ in fat amount, the patient does not necessarily have the extracellular fluid volume of 200 mL per kilogram of the actual body weight and does not necessarily have the intracellular fluid volume of 400 mL per kilogram of the actual body weight.

Considering such difference in fat amount between the healthy person in standard body shape and the patient or difference in fat amount between patients, in the present embodiment, the calculated post-hemodialysis extracellular fluid volume $_{ecf}Ve$ is divided by the ideal body weight, not by the actual body weight, to calculate a post-hemodialysis extracellular fluid volume per kilogram of the ideal body weight, and the calculated post-hemodialysis intracellular fluid volume $_{icf}Ve$ is also divided by the ideal body weight to calculate a post-hemodialysis intracellular fluid volume per kilogram of the ideal body weight.

The ideal body weight can be calculated by multiplying the square of body height (m) by 22. The calculation unit 20 calculates a post-hemodialysis extracellular fluid volume per kilogram of the ideal body weight (which may be referred to as "post-hemodialysis normalized extracellular fluid volume", hereinbelow) using the formula of Mathematical 40 below. Here, nECVe represents post-hemodialysis normalized extracellular fluid volume (mL/kg) and IBW represents ideal body weight (kg).

[Mathematical 40]

$$nECVe = \frac{_{ecf}Ve}{IBW}$$

Further, the calculation unit 20 calculates a post-hemodialysis intracellular fluid volume per kilogram of the ideal body weight (which may be referred to as "post-hemodialysis normalized intracellular fluid volume", hereinbelow) using the formula of Mathematical 41 below. Here, nICVe represents post-hemodialysis normalized intracellular fluid volume (mL/kg).

[Mathematical 41]

$$nICVe = \frac{_{icf}Ve}{IBW}$$

The body shape of hemodialysis patient is often different from that of healthy person due to not only the difference in fat amount but also the difference in muscle mass. Muscle mass changes depending on thickening or wasting of individual muscle cells. Thickening of muscle cells inevitably results in an increase in the intracellular fluid volume by a degree of the muscle thickening as well as an increase in the extracellular fluid volume surrounding the muscle cells. On the other hand, wasting of muscle cells inevitably results in a decrease in the intracellular fluid volume by a degree of the muscle wasting as well as a decrease in the extracellular fluid volume surrounding the muscle cells. Thus, if the patient has greater muscle mass than the healthy person in standard body shape, his/her post-hemodialysis normalized intracellular fluid volume nICVe is greater than the intracellular fluid volume of 400 mL/kg of the healthy person in standard body shape and his/her post-hemodialysis normalized extracellular fluid volume nECVe is also greater than the extracellular fluid volume of 200 mL/kg of the healthy person in standard body shape. On the other hand, if the patient has less muscle mass than the healthy person in standard body shape, his/her post-hemodialysis normalized intracellular fluid volume nICVe is less than the intracellular fluid volume of 400 mL/kg of the healthy person in standard body shape and his/her post-hemodialysis normalized extracellular fluid volume nECVe is also less than the extracellular fluid volume of 200 mL/kg of the healthy person in standard body shape.

The extracellular fluid volume of hemodialysis patient increases not only when the muscle mass increases but also when water excessively accumulates in the body of the patient, and it decreases not only when the muscle mass decreases but also when water is insufficient in the body of the patient. On the contrary, the intracellular fluid volume changes only when the muscle mass changes since it is not affected by excess and deficiency of the extracellular fluid volume.

Although the extracellular fluid volume of hemodialysis patient increases when he/she excessively drinks water as well as when the muscle mass increases, only the increased volume of extracellular fluid volume by the excessive drinking is responsible for blood pressure elevation and burden on the heart. Similarly, although the extracellular fluid volume of hemodialysis patient decreases when water is excessively removed by hemodialysis as well as when the muscle mass decreases, only the decreased volume of the extracellular fluid volume by the excessive water removal by hemodialysis is responsible for blood pressure drop and decrease in blood supply to the various organs. Thus, the calculation unit 20 corrects the calculated post-hemodialysis normalized extracellular fluid volume nECVe based on the pre-hemodialysis normalized intracellular fluid volume nICVe calculated in step S24 (S26). A post-hemodialysis normalized extracellular fluid volume nECVe corrected based on the pre-hemodialysis normalized intracellular fluid volume nICVe can be theoretically or experimentally acquired. Hereinbelow, a method for experimentally acquiring a corrected post-hemodialysis normalized extracellular fluid volume nECVe will be described as an example of method for correcting a post-hemodialysis normalized extracellular fluid volume nECVe based on a pre-hemodialysis normalized intracellular fluid volume nICVe.

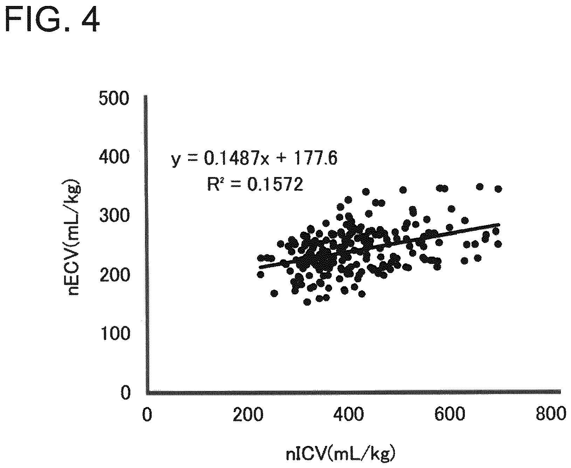

In an investigation involving 227 hemodialysis patients whose dry weights were determined as appropriate by their doctors based on the clinical symptoms, a significant correlation was found, as shown in FIG. 4 and expressed in Mathematical 42 below, between the post-hemodialysis normalized intracellular fluid volume nICVe and the post-hemodialysis normalized extracellular fluid volume nECVe.

[Mathematical 42]

$$nECVe = 0.1487 \times nICVe + 177.6 \qquad (r = 0.45; P<0.0001)$$

This result indicates that a change of I mL/kg in the post-hemodialysis normalized intracellular fluid volume nICVe causes a change of 0.15 mL/kg in the post-hemodialysis normalized extracellular fluid volume nECVe. As mentioned, a healthy person in standard body shape has intracellular fluid volume of approximately 400 mL per kilogram of the body weight. Thus, a post-hemodialysis normalized extracellular fluid volume that is to be obtained if the patient's muscle mass were equal to that of the healthy person in standard body shape, that is, a corrected post-hemodialysis normalized extracellular fluid volume is calculated by adding the post-hemodialysis normalized extracellular fluid volume nECVe of the patient to a value that is obtained by multiplying 0.15 mL/kg by a difference between 400 mL/kg, which is the intracellular fluid volume of healthy person in standard body shape, and the actual post-hemodialysis normalized intracellular fluid volume nICVe. Thus, the formula of Mathematical 43 below holds up. Here, corrected nECVe represents corrected post-hemodialysis normalized extracellular fluid volume.

[Mathematical 43]

$$\text{Corrected } nECVe = 0.15 \times (400 - nICVe) + nECVe$$

The corrected nECVe is a post-hemodialysis normalized extracellular fluid volume that is to be obtained if the muscle mass of the patient were equal to that of the healthy person in standard body shape. Thus, when the corrected nECVe is approximately 200 mL/kg, the post-hemodialysis extracellular fluid volume of the patient can be determined as appropriate, regardless of magnitude of the muscle mass. On the other hand, when the corrected nECVe is far from 200 mL/kg, the post-hemodialysis extracellular fluid volume of the patient can be determined as inappropriate.

(Experimental Example 1)

According to a clinical experiment conducted by the inventors, it has been confirmed that whether the extracellular fluid volume of a patient is appropriate or not can be assessed by using the post-hemodialysis normalized extracellular fluid volume that is calculated by the method for calculating extracellular fluid volume employed in the extracellular fluid volume calculator 10.

The body weights of patients are adjusted to, through trial and error of their doctors, weights with which edema and/or high blood pressure does not occur out of hemodialysis, the blood pressure does not drop during hemodialysis, or clinical symptoms such as fatigue and muscle cramps do not occur after hemodialysis. However, pulmonary edema may infrequently occur in some patients due to a significant increase especially in their pre-hemodialysis extracellular fluid volume caused by excessive water accumulation in their bodies. There are also patients who decline upward adjustment of their dry weights because they have a history of dissecting aneurysm, they have brain aneurysm, their previous doctors emphasized benefits of low dry weight, they read benefits of low dry weigh in books, and the like, despite that their doctors suggest the upward adjustment of the dry weights because their blood pressure dropped during hemodialysis or clinical symptoms indicative of underhydration, such as fatigue and muscle cramps, occurred after hemodialysis.

In the experiment, patients who have pulmonary edema were grouped into an overhydration group, patients who recognize their body weights are adjusted to appropriate ones by their doctors were grouped into a normohydration group, patients who decline upward adjustment of their dry weights despite underhydration being observed were grouped into a underhydration group, and the normalized extracellular fluid volumes of these patients were calculated using the method for calculating extracellular fluid volume employed in the above-described extracellular fluid volume calculator 10. That is, their post-hemodialysis extracellular fluid volumes $_{ecf}$Ve were calculated by carrying out the above-described steps S12 to S20, these extracellular fluid volumes $_{ecf}$Ve were converted to extracellular fluid volumes per kilogram of the ideal body weight (normalized extracellular fluid volumes; nECVe) by carrying out the above-described steps S22 to S24, and then these normalized extracellular fluid volumes were corrected with the normalized intracellular fluid volumes to calculate corrected normalized extracellular fluid volumes (corrected nECVe) by carrying out step S26. According to clinical records of the patients, pulmonary edema occurred in four of them in the last three years. Thus, these patients at the time when pulmonary edema occurred were grouped into the overhydration group. Further, at the time when the technique disclosed herein was completed, 10 of the patients were considered underhydrated (underhydration group) and 227 of them were considered normohydrated (normohydration group).

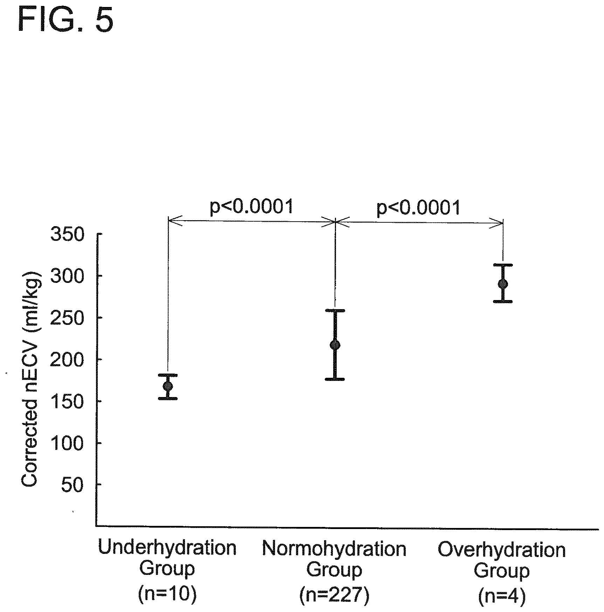

FIG. 5 shows a comparison result among corrected nECVe of the underhydration group, corrected nECVe of the normohydration group, and corrected nECVe of the overhydration group. As shown in FIG. 5, the corrected nECVe of the normohydration group was 220.1 ±39.3 mL/kg (the uncorrected nECVe (i.e., the normalized extracellular fluid volume nEVCe that was obtained by carrying out steps S12 to S24 but was not subjected to step S26) was 215.0±40.6 mL/kg (not shown)). That is, it was confirmed that in the normohydration group, the post-hemodialysis normalized extracellular fluid volume (nECVe) was close to 200 mL/kg, which is the extracellular fluid volume per kilogram of the body weight of healthy person described in a document (Hall, John (2011). Guyton and Hall textbook of medical physiology (12th ed.). Philadelphia, Pa.: Saunders/Elsevier. pp.286-287), regardless of whether it was corrected or not.

On the contrary, as shown in FIG. 5, the corrected nECVe of the underhydration group was 167.3±13.4 mL/kg (the uncorrected nEVCe was 187.3 ±19.7 mL/kg (not shown)). Further, the corrected nECVe of the overhydration group was 295.1 ±22.0 mL/kg (the uncorrected nEVCe was 297.5±30.8 mL/kg (not shown)). That is, in the underhydration group, the corrected post-hemodialysis normalized extracellular fluid volume (corrected nECVe) was less than 200 mL/kg, which is the extracellular fluid volume per kilogram of the body weight of healthy person described in the document, while in the overhydration group, the normalized extracellular fluid volume (nECVe) was greater than 200 mL/kg, which is the extracellular fluid volume per kilogram of the body weight of healthy person described in the document, regardless of whether it was corrected or not. Accordingly, it has been confirmed that whether the extracellular fluid volume of a patient is appropriate or not can be assessed by using the post-hemodialysis normalized extracellular fluid volume calculated by the method for calculating extracellular fluid volume employed in the extracellular fluid volume calculator 10.

(Experimental Example 2)

In addition, in an experiment conducted by the inventors, it has been confirmed that the muscle mass of a hemodialysis patient can be assessed by calculating his/her intracellular fluid volume. As described above, the intracellular fluid volume changes in the same manner as the muscle mass changes. Thus, it can be estimated that the muscle mass of a hemodialysis patient has decreased when his/her intracellular fluid volume has decreased despite the body weight having not changed. That is, calculating intracellular fluid volume enables sarcopenia (Masafumi KUZUYA: Chokoreikashakai niokeru Sarukopenia to Fureiru, Journal of the Japanese Society of Internal Medicine, 104: 2602-2607, 2015) to be detected early in the hemodialysis patient.

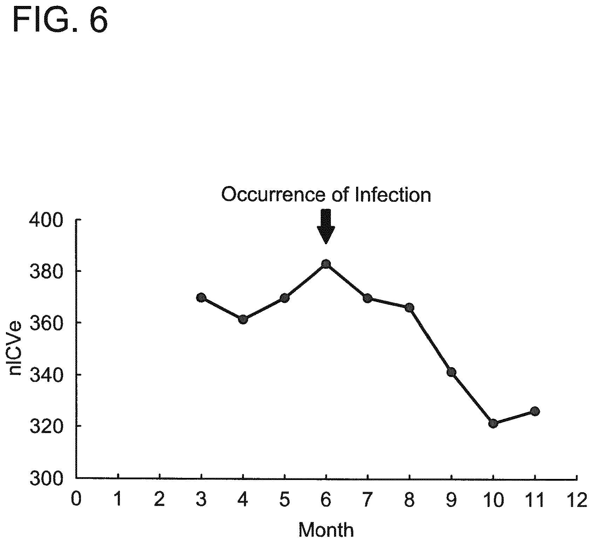

It is known that muscle mass rapidly decreases as nutritional condition gets worse (Fouque D, et al. : A proposed nomenclature and diagnostic criteria for protein-energy wasting in acute and chronic kidney disease. Kidney Int 73 :391-398, 2008). In view of this, the inventors studied how the normalized intracellular fluid volume changed in a diabetic patient who was in poor nutritional condition because the patient continuously suffered from ischemic necrosis in the left leg and infection caused by Staphylococcus aureus and lost appetite. As shown in FIG. 6, this patient got the infection caused by Staphylococcus aureus in June in addition to the ischemic necrosis in the left leg, and the normalized intracellular fluid volume started to decrease from the month. That is, the normalized intracellular fluid volume decreased as the nutritional condition of the patient got worse. Accordingly, it was confirmed that the muscle mass of hemodialysis patient can be assessed by calculating the normalized intracellular fluid volume.

In the embodiment, the post-hemodialysis extracellular fluid volume $_{ecf}Ve$ is calculated taking the water volume $_{cell}EW$ transferred from the extracellular compartment 50 to the intracellular compartment 40 during hemodialysis into consideration, however, the water volume $_{cell}EW$ transferred from the extracellular compartment 50 to the intracellular compartment 40 during hemodialysis may not be took into consideration. Water volume transferred to the outside of the extracellular compartment 50 during hemodialysis includes the removal volume of water $_{dial}EW$ removed to the outside of body by hemodialysis and the water volume $_{cell}EW$ transferred from the extracellular compartment 50 to the intracellular compartment 40 during hemodialysis, and taking these water volumes into consideration results in more accurate calculation of the post-hemodialysis extracellular fluid volume $_{ecf}Ve$. That said, the water volume $_{cell}EW$ transferred to the intracellular compartment 40 is insignificant as compared to the removal volume of water $_{dial}EW$ removed by hemodialysis, thus the post-hemodialysis extracellular fluid volume $_{ecf}Ve$ is calculated almost accurately even when the water volume $_{cell}EW$ transferred to the intracellular compartment 40 is considered as zero. Thus, the post-hemodialysis extracellular fluid volume $_{ecf}Ve$ may be calculated with the water volume $_{cell}EW$ transferred to the intracellular compartment 40 considered as zero. In this case, zero is substituted, as the water volume $_{cell}EW$ transferred to the intracellular compartment 40, into the above formula of Mathematical 3. Then, in step S20, the calculation unit 20 substitutes the pre-hemodialysis and post-hemodialysis uric acid concentrations $_{acid}Cs$ and $_{acid}Ce$ stored in the patient's information storage unit 14, the removal volume of water $_{dial}EW$ stored in the hemodialysis information storage unit 16, and the removal amount of uric acid $_{acid}E$ calculated in step S18 into the formula of Mathematical 3, to calculate the post-hemodialysis extracellular fluid volume $_{ecf}Ve$.

In the embodiment, a measured value is used as the post-hemodialysis hematocrit value, however, the post-hemodialysis hematocrit value may be calculated from a measured pre-hemodialysis hematocrit value, the patient's body weight, and removal volume of water, and the calculated hematocrit value may be used. The post-hemodialysis hematocrit value can be obtained theoretically or experimentally. Hereinbelow, a method for experimentally calculating a post-hemodialysis hematocrit value based on pre-hemodialysis hematocrit value will be described as an exemplary method for calculating a post-hemodialysis hematocrit value. As a result of analysis on data of 30 hemodialysis patients, it was found that there is the relationship below between a ratio of body weight difference before and after hemodialysis to the ideal body weight and a ratio of post-hemodialysis hematocrit value to pre-hemodialysis hematocrit value. Here, BWs represents pre-hemodialysis body weight and BWe represents post-hemodialysis body weight.

[Mathematical 44]

$$\frac{Hte}{Hts} = 2.8137284 \times \frac{BWs - BWe}{IBW} + 0.9661694$$

By rearranging the formula of Mathematical 44, a formula for calculating the post-hemodialysis hematocrit value based on the pre-hemodialysis hematocrit value, the ideal body weight, and the pre-hemodialysis and post-hemodialysis body weights is obtained.

[Mathematical 45]

$$Hte = (2.8137284 \times \frac{BWs - BWe}{IBW} + 0.9661694) \times Hts$$

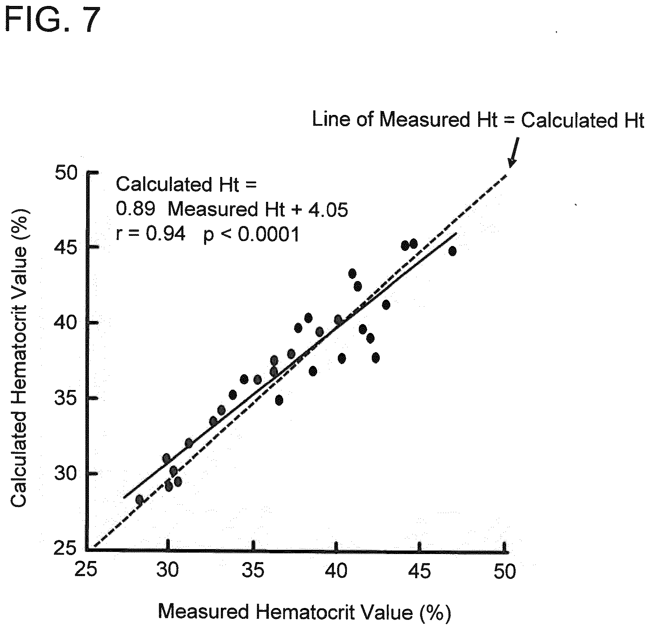

For other 30 hemodialysis patients than the above-mentioned 30 patients, their post-hemodialysis hematocrit values calculated using Mathematical 45 were compared to their measured post-hemodialysis hematocrit values. As shown in FIG. 7, the post-hemodialysis hematocrit values calculated using Mathematical 45 substantially match the measured post-hemodialysis hematocrit values. Accordingly, it was confirmed that the post-hemodialysis hematocrit value can be calculated, using the above Mathematical 45, from the pre-hemodialysis hematocrit value, the ideal body weight, and the pre-hemodialysis and post-hemodialysis body weights.

In the embodiment, the removal volume of water $_{dial}EW$ is inputted by the operator into the processor 12, however, it is not limited thereto. For example, the processor 12 may be configured to receive input of pre-hemodialysis and post-hemodialysis body weights of a patient, and calculate the removal volume of water $_{dial}EW$ based on the pre-hemodialysis and post-hemodialysis body weights of the patient.

In the embodiment, the post-hemodialysis extracellular fluid volume is corrected using the post-hemodialysis normalized intracellular fluid volume nICV, however, it is not limited thereto. The post-hemodialysis extracellular fluid volume $_{ecf}Ve$ calculated in step S20 can be used to assess whether the extracellular fluid volume that remains in the body of patient after hemodialysis is appropriate or not.

In the embodiment, the post-hemodialysis extracellular fluid volume is calculated, however, a pre-hemodialysis extracellular fluid volume may be calculated in addition to the post-hemodialysis extracellular fluid volume. For a patient whose pre-hemodialysis body weight significantly increases due to excessive water drinking out of hemodialysis, an upper limit of the water volume the patient is allowed to drink can be determined by calculating the pre-hemodialysis extracellular fluid volume.

In the embodiment, the post-hemodialysis normalized intracellular fluid volume is calculated and this is used to correct the post-hemodialysis normalized extracellular fluid volume, however, it is not limited thereto. For example, a pre-hemodialysis normalized intracellular fluid volume may be calculated instead of the post-hemodialysis normalized intracellular fluid volume, and that may be used to correct the post-hemodialysis normalized extracellular fluid volume. The pre-hemodialysis normalized intracellular fluid volume is not affected by change in the serum sodium concentration caused by hemodialysis treatment. Thus, the post-hemodialysis normalized extracellular fluid volume can be accurately corrected even using the pre-hemodialysis normalized intracellular fluid volume instead of the post-hemodialysis normalized intracellular fluid volume.

In the embodiment, the post-hemodialysis extracellular fluid volume $_{ecf}Ve$ is calculated based on the difference between the pre-hemodialysis and post-hemodialysis uric acid quantities, however, it is not limited thereto. Any substance that is incapable of passing through the cell membrane 42 and capable of passing through the capillary membrane 56 may be used, and the post-hemodialysis extracellular fluid volume $_{ecf}Ve$ may be calculated, for example, based on a difference of β2-microglobulin.

While specific examples of the present disclosure have been described above in detail, these examples are merely illustrative and place no limitation on the scope of the patent claims.

**Claims**

1. An extracellular fluid volume calculator (10) comprising:

   a processor (12); and
   a memory storing computer-readable instructions therein,
   wherein the computer-readable instructions, when executed by the processor (12), cause the extracellular fluid volume calculator (10) to execute
   acquiring a pre-hemodialysis substance concentration, a post-hemodialysis substance concentration, a removal amount of a substance by hemodialysis, and a removal volume of water during hemodialysis, wherein the pre-hemodialysis substance concentration is a pre-hemodialysis concentration of the substance in plasma, the post-hemodialysis substance concentration is a post-hemodialysis concentration of the substance in plasma, and the substance is incapable of passing through a cell membrane (42) and is capable of passing through a capillary membrane (56); and
   calculating an extracellular fluid volume based on a difference between a pre-hemodialysis amount of the substance and a post-hemodialysis amount of the substance, the difference being determined based on the pre-hemodialysis substance concentration, the post-hemodialysis substance concentration, the removal amount of substance, and the removal volume of water.

2. The extracellular fluid volume calculator (10) according to claim 1, wherein

   the substance is a uric acid,
   the pre-hemodialysis substance concentration is a pre-hemodialysis plasma uric acid concentration,
   the post-hemodialysis substance concentration is a post-hemodialysis plasma uric acid concentration, and
   the removal amount of substance is a removal amount of uric acid by hemodialysis.

3. The extracellular fluid volume calculator (10) according to claim 2, wherein
   the computer-readable instructions, when executed by the processor (12), cause the extracellular fluid volume calculator (10) to further execute:

   acquiring a dialyzer mass transfer-area coefficient for uric acid, a blood flow rate through a dialyzer, a dialysate flow rate through the dialyzer, and a hematocrit value;
   calculating a dialyzer clearance for uric acid based on the dialyzer overall mass transfer-area coefficient for uric acid, the blood flow rate, the dialysate flow rate, and the hematocrit value; and
   calculating the removal amount of uric acid based on the calculated dialyzer clearance for uric acid, the pre-hemodialysis plasma uric acid concentration, and the post-hemodialysis plasma uric acid concentration.

4. The extracellular fluid volume calculator (10) according to any one of claims 1 to 3, wherein
   the computer-readable instructions, when executed by the processor (12), cause the extracellular fluid volume calculator (10) to further execute:

   acquiring a pre-hemodialysis plasma urea concentration, a post-hemodialysis plasma urea concentration, and a removal amount of urea by hemodialysis; and
   calculating a total body fluid volume in a body based on the pre-hemodialysis plasma urea concentration, the post-hemodialysis plasma urea concentration, the removal amount of urea and the removal volume of water.

5. The extracellular fluid volume calculator (10) according to claim 4, wherein the computer-readable instructions, when executed by the processor (12), cause the extracellular fluid volume calculator (10) to further execute calculating an intracellular fluid volume by subtracting the calculated extracellular fluid volume from the calculated total body fluid volume in the body.

6. The extracellular fluid volume calculator (10) according to claim 5, wherein the computer-readable instructions, when executed by the processor (12), cause the extracellular fluid volume calculator (10) to further execute correcting the extracellular fluid volume by using the calculated intracellular fluid volume.

7. A method for calculating an extracellular fluid volume, the method comprising:

acquiring a pre-hemodialysis plasma uric acid concentration;
acquiring a post-hemodialysis plasma uric acid concentration;
acquiring a removal amount of uric acid by hemodialysis and a removal volume of water during hemodialysis; and
calculating a post-hemodialysis extracellular fluid volume based on a difference between a pre-hemodialysis amount of uric acid and a post-hemodialysis amount of uric acid, the difference being determined based on the pre-hemodialysis plasma uric acid concentration, the post-hemodialysis plasma uric acid concentration, the removal amount of uric acid, and the removal volume of water.

**Patentansprüche**

1. Rechner (10) für extrazelluläres Flüssigkeitsvolumen, der Folgendes umfasst:

einen Prozessor (12); und
einen Speicher, in dem computerlesbare Anweisungen gespeichert sind,
wobei die computerlesbaren Anweisungen, wenn sie von dem Prozessor (12) ausgeführt werden, den Rechner (10) für extrazelluläres Flüssigkeitsvolumen veranlassen, Folgendes auszuführen
Erfassen einer Substanzkonzentration vor der Hämodialyse, einer Substanzkonzentration nach der Hämodialyse, einer Entfernungsmenge einer Substanz durch Hämodialyse und eines Entfernungsvolumens von Wasser während der Hämodialyse, wobei die Substanzkonzentration vor der Hämodialyse eine Konzentration der Substanz vor der Hämodialyse im Plasma ist, die Substanzkonzentration nach der Hämodialyse eine Konzentration der Substanz nach der Hämodialyse im Plasma ist, und die Substanz nicht in der Lage ist, eine Zellmembran (42) zu passieren, und in der Lage ist, eine Kapillarmembran (56) zu passieren; und
Berechnen eines extrazellulären Flüssigkeitsvolumens basierend auf einer Differenz zwischen einer Menge der Substanz vor der Hämodialyse und einer Menge der Substanz nach der Hämodialyse, wobei die Differenz basierend auf der Substanzkonzentration vor der Hämodialyse, der Substanzkonzentration nach der Hämodialyse, der Entfernungsmenge der Substanz und des Entfernungsvolumens des Wassers bestimmt wird.

2. Rechner (10) für extrazelluläres Flüssigkeitsvolumen nach Anspruch 1, wobei

die Substanz eine Harnsäure ist,
die Substanzkonzentration vor der Hämodialyse eine Plasmaharnsäurekonzentration vor der Hämodialyse ist,
die Substanzkonzentration nach der Hämodialyse eine Plasmaharnsäurekonzentration nach der Hämodialyse ist, und
die Entfernungsmenge der Substanz die Menge der durch Hämodialyse entfernten Harnsäure ist.

3. Rechner (10) für extrazelluläres Flüssigkeitsvolumen nach Anspruch 2, wobei die computerlesbaren Anweisungen, wenn sie von dem Prozessor (12) ausgeführt werden, den Rechner (10) für extrazelluläres Flüssigkeitsvolumen veranlassen, ferner Folgendes auszuführen:

Erfassen eines Dialysator-Massentransfer-Flächenkoeffizienten für Harnsäure, einer Blutflussrate durch einen Dialysator, einer Dialysatflussrate durch den Dialysator und eines Hämatokritwertes;
Berechnen einer Dialysator-Clearance für Harnsäure basierend auf dem Gesamt-Dialysator-Massentransfer-Flächenkoeffizienten für Harnsäure, der Blutdurchflussrate, der Dialysatdurchflussrate und des Hämatokritwerts; und
Berechnen der entfernten Harnsäuremenge basierend auf der berechneten Dialysator-Clearance für Harnsäure, der Plasmaharnsäurekonzentration vor der Hämodialyse und der Plasmaharnsäurekonzentration nach der Hämodialyse.

4. Rechner (10) für extrazelluläres Flüssigkeitsvolumen nach einem der Ansprüche 1 bis 3, wobei die computerlesbaren Anweisungen, wenn sie von dem Prozessor (12) ausgeführt werden, den Rechner (10) für extrazelluläres Flüssigkeitsvolumen veranlassen, ferner Folgendes auszuführen:

Erfassen einer Plasmaharnstoffkonzentration vor der Hämodialyse, einer Plasmaharnstoffkonzentration nach der Hämodialyse und einer durch Hämodialyse entfernten Menge an Harnstoff; und
Berechnen eines Gesamtkörperflüssigkeitsvolumens in einem Körper basierend auf der Plasmaharnstoffkonzentration vor der Hämodialyse, der Plasmaharnstoffkonzentration nach der Hämodialyse, der Entfernungsmenge von Harnstoff und des Entfernungsvolumens von Wasser.

5. Rechner (10) für extrazelluläres Flüssigkeitsvolumen nach Anspruch 4, wobei die computerlesbaren Anweisungen, wenn sie von dem Prozessor (12) ausgeführt werden, den Rechner (10) für extrazelluläres Flüssigkeitsvolumen veranlassen, ferner die Berechnung eines intrazellulären Flüssigkeitsvolumens durch Subtraktion des berechneten extrazellulären Flüssigkeitsvolumens von dem berechneten Gesamtvolumen der Körperflüssigkeit im Körper auszuführen.

6. Rechner (10) für extrazelluläres Flüssigkeitsvolumen nach Anspruch 5, wobei die computerlesbaren Anweisungen, wenn sie von dem Prozessor (12) ausgeführt werden, den Rechner (10) für extrazelluläres Flüssigkeitsvolumen veranlassen, das extrazelluläre Flüssigkeitsvolumen unter Verwendung des berechneten intrazellulären Flüssigkeitsvolumens ferner zu korrigieren.

7. Verfahren zum Berechnen eines extrazellulären Flüssigkeitsvolumens, wobei das Verfahren Folgendes umfasst:

Erfassen einer Plasmaharnsäurekonzentration vor der Hämodialyse;
Erfassen einer Plasmaharnsäurekonzentration nach der Hämodialyse;
Erfassen einer durch Hämodialyse entfernten Harnsäuremenge und eines Entfernungsvolumens von Wasser während der Hämodialyse; und
Berechnen eines extrazellulären Flüssigkeitsvolumens nach der Hämodialyse basierend auf einer Differenz zwischen einer Harnsäuremenge vor der Hämodialyse und einer Harnsäuremenge nach der Hämodialyse, wobei die Differenz basierend auf der Plasmaharnsäurekonzentration vor der Hämodialyse, der Plasmaharnsäurekonzentration nach der Hämodialyse, der Entfernungsmenge von Harnsäure und des Entfernungsvolumens von Wasser bestimmt wird.

## Revendications

1. Calculateur de volume de fluide extracellulaire (10) comprenant :

un processeur (12) ; et
une mémoire stockant des instructions lisibles par ordinateur,
dans lequel les instructions lisibles par ordinateur, lorsqu'elles sont exécutées par le processeur (12), amènent le calculateur de volume de fluide extracellulaire (10) à exécuter l'acquisition d'une concentration de substance de pré-hémodialyse, d'une concentration de substance de post-hémodialyse, d'une quantité d'élimination d'une substance par hémodialyse, et
d'un volume d'élimination d'eau pendant l'hémodialyse, dans laquelle la concentration de substance de pré-hémodialyse est une concentration de pré-hémodialyse de la substance dans le plasma, la concentration de substance de post-hémodialyse est une concentration de post-hémodialyse de la substance dans le plasma, et la substance est incapable de traverser une membrane cellulaire (42) et est capable de traverser une membrane capillaire (56) ; et
le calcul d'un volume de fluide extracellulaire sur la base d'une différence entre une quantité pré-hémodialyse de la substance et une quantité post-hémodialyse de la substance, la différence étant déterminée sur la base de la concentration de substance pré-hémodialyse, la concentration de substance post-hémodialyse, la quantité d'élimination de substance et le volume d'élimination d'eau.

2. Calculateur de volume de fluide extracellulaire (10) selon la revendication 1, dans lequel

la substance est un acide urique,
la concentration de substance pré-hémodialyse est une concentration d'acide urique plasmatique pré-hémodialyse,
la concentration de substance post-hémodialyse est une concentration d'acide urique plasmatique post-hémodialyse, et
la quantité d'élimination de substance est une quantité d'élimination d'acide urique par hémodialyse.

3. Calculateur de volume de fluide extracellulaire (10) selon la revendication 2, dans lequel les instructions lisibles par ordinateur, lorsqu'elles sont exécutées par le processeur (12), amènent le calculateur de volume de fluide extracellulaire (10) à exécuter en outre :

l'acquisition d'un coefficient de surface de transfert de masse de dialyseur pour l'acide urique, un débit sanguin à travers un dialyseur, un débit de dialysat à travers le dialyseur et une valeur d'hématocrite ;
le calcul d'une clairance du dialyseur pour l'acide urique sur la base du coefficient global de surface de transfert de masse de dialyseur pour l'acide urique, du débit sanguin, du débit du dialysat
et de la valeur d'hématocrite ; et
le calcul de la quantité d'élimination d'acide urique sur la base de la clairance du dialyseur calculée pour l'acide urique, la concentration d'acide urique plasmatique pré-hémodialyse et la concentration d'acide urique plasmatique post-hémodialyse.

4. Calculateur de volume de fluide extracellulaire (10) selon l'une quelconque des revendications 1 à 3, dans lequel les instructions lisibles par ordinateur, lorsqu'elles sont exécutées par le processeur (12), amènent le calculateur de volume de fluide extracellulaire (10) à exécuter en outre :

l'acquisition d'une concentration d'urée plasmatique pré-hémodialyse, d'une concentration d'urée plasmatique post-hémodialyse et d'une quantité d'élimination d'urée par hémodialyse ; et
le calcul d'un volume de fluide corporel total dans un corps sur la base de la concentration d'urée plasmatique pré-hémodialyse, de la concentration d'urée plasmatique post-hémodialyse, de la quantité d'élimination d'urée et du volume d'élimination d'eau.

5. Calculateur de volume de fluide extracellulaire (10) selon la revendication 4, dans lequel les instructions lisibles par ordinateur, lorsqu'elles sont exécutées par le processeur (12), amènent le calculateur de volume de fluide extracellulaire (10) à exécuter en outre le calcul d'un volume de fluide intracellulaire en soustrayant le volume de fluide extracellulaire calculé du volume de fluide corporel total calculé dans le corps.

6. Calculateur de volume de fluide extracellulaire (10) selon la revendication 5, dans lequel les instructions lisibles par ordinateur, lorsqu'elles sont exécutées par le processeur (12), amènent le calculateur de volume de fluide extracellulaire (10) à exécuter en outre la correction du volume de fluide extracellulaire en utilisant le volume de fluide intracellulaire calculé.

7. Procédé de calcul d'un volume de fluide extracellulaire, le procédé comprenant :

l'acquisition d'une concentration plasmatique d'acide urique pré-hémodialyse ;
l'acquisition d'une concentration plasmatique d'acide urique post-hémodialyse ;
l'acquisition d'une quantité d'élimination d'acide urique par hémodialyse et d'un volume d'élimination d'eau pendant l'hémodialyse ; et
le calcul d'un volume de fluide extracellulaire post-hémodialyse sur la base d'une différence entre une quantité d'acide urique pré-hémodialyse et une quantité d'acide urique post-hémodialyse, la différence étant déterminée sur la base de la concentration plasmatique d'acide urique pré-hémodialyse, la concentration d'acide urique plasmatique post-hémodialyse, la quantité d'élimination d'acide urique, et le volume d'élimination d'eau.

# FIG. 1

# FIG. 2

# FIG. 3

```
                    ┌─────────────┐
                    │    Start    │
                    └──────┬──────┘
                           │                              S12
                           ▼
┌──────────────────────────────────────────────────────────┐
│ Acquire Various Types of Information About Hemodialysis Patient │
└──────────────────────────┬───────────────────────────────┘
                           │                              S14
                           ▼
  ┌────────────────────────────────────────────────────────┐
  │ Acquire Various Types of Information About Hemodialysis │
  └───────────────────────┬────────────────────────────────┘
                          │                               S16
                          ▼
   ┌──────────────────────────────────────────────────────┐
   │ Calculate Post-Hemodialysis Total Body Fluid Volume   │
   └──────────────────────┬───────────────────────────────┘
                          │                               S18
                          ▼
     ┌──────────────────────────────────────────────┐
     │ Calculate Removal Amount of Uric Acid         │
     └─────────────────────┬────────────────────────┘
                           │                            S20
                           ▼
  ┌──────────────────────────────────────────────────────┐
  │ Calculate Post-Hemodialysis Extracellular Fluid Volume │
  └──────────────────────┬───────────────────────────────┘
                         │                                S22
                         ▼
 ┌───────────────────────────────────────────────────────┐
 │ Calculate Post-Hemodialysis Intracellular Fluid Volume │
 └──────────────────────┬────────────────────────────────┘
                        │                                 S24
                        ▼
┌────────────────────────────────────────────────────────┐
│ Calculate Normalized Extracellular Fluid Volume and     │
│ Normalized Intracellular Fluid Volume                   │
└──────────────────────┬─────────────────────────────────┘
                       │                                  S26
                       ▼
┌────────────────────────────────────────────────────────┐
│ Correct Normalized Extracellular Fluid Volume           │
│ Using Normalized Intracellular Fluid Volume             │
└──────────────────────┬─────────────────────────────────┘
                       │
                       ▼
                ┌─────────────┐
                │     End     │
                └─────────────┘
```

# FIG. 4

# FIG. 5

# FIG. 6

# FIG. 7

Line of Measured Ht = Calculated Ht

Calculated Ht =
0.89 Measured Ht + 4.05
r = 0.94   p < 0.0001

Calculated Hematocrit Value (%)

Measured Hematocrit Value (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2594300 A1

### Non-patent literature cited in the description

- **LUIK A et al.** Blood pressure control and fluid state in patients on long treatment time dialysis. *J Am Soc Nephrol,* 1994, vol. 5, 521
- **JAEGER JQ ; MEHTA RL.** Assessment of Dry Weight in Hemodialysis: An Overview. *J Am Soc Nephrol,* 1999, vol. 10, 392-403
- **CHARRA B et al.** Clinical assessment of dry weight. *Nephrol Dial Transplant,* 1996, vol. 11 (2), 16-19
- **JAEGER JQ ; MEHTA RL.** Assessment of Dry Weight in Hemodialysis: An Overview. *J Am Soc Nephrol,* 1999, vol. 10, 392-403
- **GUNAL AI.** How to determine 'dry weight'?. *Kidney Int,* 2013, vol. 3, 377-379
- **GUNAL AL.** How to determine 'dry weight'?. *Kidney Int,* 2013, vol. 3, 377-379
- **ERIKO ISHII et al.** The target range of plasma ANP level for dry weight adjustment in HD patients. *Journal of Japanese Society for Dialysis Therapy,* 2004, vol. 37, 1417-1422
- **BRANDT RR et al.** Atrial natriuretic peptide in heart failure. *J Am Coll CardioL,* 1993, vol. 22 (A), 86A-92A
- **NAGENDRA S et al.** A comparative study of plasma uric acid, erythrocyte uric acid and urine uric acid levels in type 2 diabetic subjects. *Merit Research Journal,* 2015, vol. 3, 571-574
- **ERIC DESCOMBES et al.** Diffusion kinetics of urea, creatinine and uric acid in blood during hemodialysis. Clinical implications. *Clinical Nephrology,* 1993, vol. 40, 286-295
- **HALL, JOHN.** Guyton and Hall textbook of medical physiology. Saunders/Elsevier, 2011, 286-287
- **MASAFUMI KUZUYA.** Chokoreikashakai niokeru Sarukopenia to Fureiru. *Journal of the Japanese Society of Internal Medicine,* 2015, vol. 104, 2602-2607
- **FOUQUE D et al.** A proposed nomenclature and diagnostic criteria for protein-energy wasting in acute and chronic kidney disease. *Kidney Int,* 2008, vol. 73, 391-398